# EUROPEAN PATENT SPECIFICATION

(11) **EP 4 037 728 B1**
(45) Date of publication and mention of the grant of the patent: **27.05.2026**
(21) Application number: 20872087.0
(22) Date of filing: 01.10.2020
(51) Int. Cl.: A61B 5/0215, A61B 5/021, A61B 5/06, A61M 5/42, A61M 5/48, A61B 5/00

(54) **DEVICES AND METHODS FOR DETERMINING THE POSITION OF AN INTRAVASCULAR PROBE**
VORRICHTUNGEN UND VERFAHREN ZUR BESTIMMUNG DER POSITION EINER INTRAVASKULÄREN SONDE
DISPOSITIFS ET PROCÉDÉS DE DÉTERMINATION DE LA POSITION D'UNE SONDE INTRAVASCULAIRE

(30) Priority: 02.10.2019 US 201962909253 P; 02.10.2019 US 201962909254 P
(43) Date of publication of application: 10.08.2022
(73) Proprietor: Angie Technologies Ltd., 6936465 Tel Aviv (IL)
(72) Inventor: GERSHI, Israel, 6936465 Tel Aviv (IL); ELMAN, Vladimir, 5837409 Holon (IL)
(74) Representative: Vossius & Partner Patentanwälte Rechtsanwälte mbB
(86) International application number: PCT/IL2020/051071
(87) International publication number: WO 2021/064732

(56) References cited:
- WO-A1-2014/007949
- US-A1- 2011 046 477
- US-A1- 2011 046 477
- US-A9- 2016 374 612

## Description

### TECHNOLOGICAL FIELD

The present invention relates generally to venipuncture, which is the procedure of obtaining access into a blood vessel of a patient. More specifically, the present invention relates to determination of the position of an intravascular probe.

### BACKGROUND ART

References considered to be relevant as background to the presently disclosed subject matter are listed below:
- US patent No. 3,785,367
- US patent No. 4,215,702
- US patent No. 5,314,410
- US patent No. 5,520,193
- US patent No. 5,954,701
- US patent No. 8,574,195
- US patent application No. 2004/0243007
- US patent application No. 2011/046477
- US patent application No.2011/0060229
- US patent application No. 2011/071479
- US patent application No.2012/0172750

Acknowledgement of the above references herein is not to be inferred as meaning that these are in any way relevant to the patentability of the presently disclosed subject matter.

### BACKGROUND

Venipuncture may be utilized, e.g., for intravenous therapy or for sampling of venous blood. In human medicine, this procedure may be performed by a medical practitioner, for example a physician, a medical laboratory scientist, an emergency medical technician (EMT), a paramedic, a phlebotomist, a dialysis technician, or other nursing staff. In veterinary medicine, the procedure may be performed by a veterinary practitioner, for example a veterinarian or a veterinary technician. Venipuncture is one of the most routinely performed invasive procedures, and may be performed in order to obtain blood for diagnostic purposes, to monitor levels of blood components, to administer therapeutic treatments including medications, nutrition, or chemotherapy, to remove blood due to excess levels of iron or erythrocytes, and/or to collect blood for later uses, e.g. for blood donation (transplantation to other person) and/or for autologous blood donation (transplantation to the same person).

Venipuncture is performed by inserting a probe, such as an intravenous catheter, into a blood vessel of the patient. For efficient performance of the procedure, the practitioner should have an indication when the probe enters and/or exits the vein. Some indication may be obtained by visual observation of the blood flowing from the vein though the needle probe to an associated reservoir (e.g., a syringe). Intravascular catheter systems, such as peripheral intravenous catheters or central venous catheters, for example, are essential in modern medical practice. Catheter systems are commonly used for fluid infusion or withdrawal, or for monitoring various physiological parameters, such as blood pressure, pH, and blood gas tensions.

The conventional method of placing the catheter into a blood vessel using over-the-needle technique, comprises skin puncture with an introducer needle, moving the needle in surrounding tissues forward towards the targeted blood vessel, puncturing the wall of a targeted blood vessel, and pushing a catheter inside the targeted blood vessel while removing the needle. In cases where the over-the-wire technique is being used, a guidewire is used prior to placement or insertion of the catheter inside of the blood vessel. An indication of a successful blood vessel puncture is a blood flow into a flashback chamber that is commonly visually observed. However, one of the major problems during venipuncture or arterial line placement is difficulty in determination of the exact position of a needle tip inside the skin relative to a blood vessel. In most conventional systems, venipuncture is performed based on the results of both visual observation and palpation of the skin area to be punctured, and often a blood vessel is not located correctly on a first try. Another problem is that an observation of blood flow to a flashback chamber is not always a reliable indicator of a blood vessel's wall penetration. An amount of blood may also appear in the flashback chamber, even in cases where the needle sufficiently penetrates the blood vessel, thus ending up not in the lumen of the blood vessel, but in surrounding tissues. Should a clinician not be able to determine that the blood vessel has been penetrated to the desirable extent catheter placement into an incorrectly punctured blood vessel may cause severe damage. This may relate to yet another problem, where the clinician may not be able to determine when precisely to stop inserting the needle into the blood vessel, which may also lead to damaging the blood vessel from within. In fact, correct positioning of a catheter system on any patient with small, deep, faulty or damaged veins, is coupled with the aforesaid problems. All of the abovementioned problems become especially critical in case of emergency, (e.g. in an ambulance), treatment of the elderly, and/or in children's hospitals.

### GENERAL DESCRIPTION

Some aspects of this disclosure involve a technique for determining the position of a distal end of a probe relative to a blood vessel of a patient, based on pressure-values correlating to pressure of fluid at the distal end. More specifically, the position of the distal end is determined by comparing the pressure-values with one or more adaptive thresholds, which are determined based on pressure-values obtained while the distal end is located inside the blood vessel. In this connection, it should be understood that some techniques (e.g. as described in US patent application No. 2011/0060229 above) have tried to determine a position of a probe relative to a blood vessel by arbitrarily determining a threshold or a range of thresholds being indicative of a certain position of a probe relative to a blood vessel. This threshold, being typically empirically defined, may be compared to a pressure value measured during the venipuncture, and if the pressure value is above/below the threshold, the position of the probe relative to the blood vessel is determined to be in/out of the blood vessel. These techniques are not accurate enough and report multiple false alarms, since the real-time pressure value depends on a plurality of personalized parameters (such as age, gender, and weight of the patient, and his body position during the procedure (e.g. lying down or sitting)) which cannot be considered by comparing the measured pressure value to the empirical threshold. Moreover, if a tourniquet is used, the ranges of venous pressures may be further expanded, especially where the tightening of the tourniquet depends on the force that the operator voluntarily activates in a certain operation. To overcome the above-mentioned problems and to be able to determine a transition state from inside the blood vessel to outside the blood vessel or *vice versa* in real-time (which, as detailed above, is critical for this type of medical procedure), a plurality of pressure values should be measured continuously to accurately identify a change in the pressure value pattern. Therefore, based on the assumption that the pressure inside the blood vessel is higher than the pressure outside the blood vessel, the present invention enables to estimate the likelihood of the distal end of a probe to be located inside or outside the blood vessel, by comparing the pressure-values with one or more thresholds, and determining the state indication accordingly (e.g., sets the state indication to the in-blood vessel state when the pressure-values exceed a certain threshold, and/or sets the state indication to the out-of-blood vessel state when the pressure-values are below a certain threshold). The threshold(s) is/are thus determined at the beginning of the procedure adaptively, based on pressure-values correlating to the actual pressure within the blood vessel, i.e. pressure-values obtained during an in-blood vessel state. Therefore, the term *"adaptive threshold"* refers hereinafter to the threshold being determined by processing a plurality of pressure values being indicative of a pressure of a fluid when the distal end of the probe is located inside the blood vessel. The adaptive threshold defines a real-time personalized value being indicative of a transition from a first position state to a second position state, or *vice versa.* The term "real-time" defines that the practitioner gets the indication signal faster than he can perceive e.g. within about less than 0.2 seconds.

According to the present invention, there is provided a device in accordance with claim 1. Further advantageous features of the device are defined in the dependent claims.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1A** is a schematic flow chart illustrating the main steps of the method for determining a position of a distal end of a probe relative to a blood vessel of a patient according to some teachings of the present invention;
**Fig. 1B** is a schematic block diagram illustrating the main position states of a distal end of a probe relative to a blood vessel of a patient and the possible transitions from state to state;
**Fig. 2** is a schematic flow chart illustrating an embodiment of a method for indicating a position of a distal end of a probe relative to a blood vessel, utilizing one or more adaptive thresholds;
**Fig. 3** schematically illustrates an embodiment of a method for indicating a position of a distal end of a probe relative to a blood vessel, utilizing tendencies of pressure-values according to some embodiments of the present invention;
**Fig. 4** schematically illustrates an embodiment of a method for indicating a position of an intravascular catheter comprising a needle and a cannula mounted over the needle;
**Fig. 5A** is a schematic block diagram illustrating the main functional part of the device for determining a position of a distal end of a probe relative to a blood vessel of a patient according to some teachings of the present invention;
**Fig. 5B** is a schematic block diagram illustrating the main functional part of the device for determining a position of a distal end of a probe relative to a blood vessel of a patient according to some teachings of the present invention;
**Fig. 6** schematically illustrates an embodiment of a device for indicating a position of a distal end of a probe relative to a blood vessel of a patient;
**Fig. 7** schematically illustrates an embodiment of a device for determining a position of a distal end of a probe relative to a blood vessel of a patient comprising *inter alia* an accelerometer;
**Fig. 8a** and **Fig. 8b** schematically illustrate an embodiment of a device for indicating a position of an intravascular catheter comprising a needle and a cannula mounted over the needle;
**Fig. 9a** and **Fig. 9b** graphically illustrate two different pattern pressures to be processed according to some teachings of the present invention;
**Fig. 10** schematically illustrates a position-indicating device for visually indicating a position of a distal end of a probe relative to a blood vessel of a patient, according to one example of the presently disclosed subject matter;
**Fig. 11** is a schematic cross-sectional view along line A-A in Fig. 10;
**Fig. 12** is an enlarged view of section A1 in Fig. 11; and
**Fig. 13** is schematic representation of the device of Fig. 10, with the housing thereof omitted for illustration purposes.

### DETAILED DESCRIPTION OF EMBODIMENTS

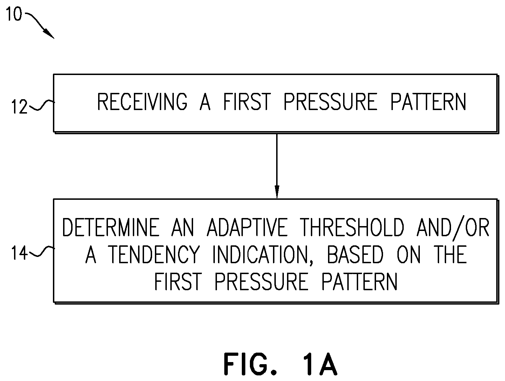

Reference is made to Fig. 1A schematically illustrating, by way of a flow chart, the main steps of a method 10 for determining a position of a distal end of a probe relative to a blood vessel of a patient according to some teachings of the present invention. The method **10** comprises receiving in **12** a first pressure pattern comprising a plurality of pressure values being indicative of a pressure of a fluid while the distal end of the probe is located inside the blood vessel and in **14** processing the first pressure pattern and determining at least one adaptive threshold and/or a tendency condition being indicative of a transition from a first position state to a second position state, or *vice versa.*

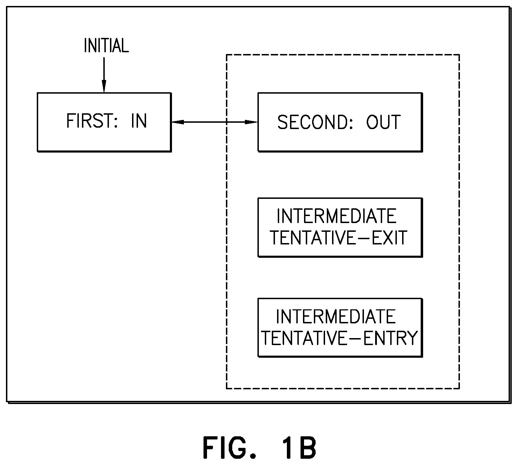

Reference is made to **Fig. 1B** schematically illustrating, by way of a block diagram, the main position states of a distal end of a probe relative to a blood vessel of a patient, and the possible transitions between them. The first position state defines an in-blood vessel condition in which the distal end of the probe is located inside the blood vessel and the second position state defines at least one exit condition in which the distal end of the probe is located at least partially outside the blood vessel. The technique also enables to determine a plurality of intermediate states being indicative of a fast change (i.e. tendency) in pressure as compared to other pressure changes in the pressure pattern. The intermediate states may be indicative of a transition process from one state to another or *vice versa.* The plurality of intermediate states comprise the following states: a first intermediate state indicative of a potential entry into the blood vessel referred also as tentative-entry state if the probe was out-of-vessel, and the second intermediate state indicative of a potential exit out of the blood vessel referred also as tentative-exit state, if the probe was in-vessel. Therefore, the second intermediate state (the tentative-exit state) initiates from the first state (i.e. from the in-vessel state).

The in-vessel state and the out-of-vessel state are referred to as stable states or steady states. The state indication may also be set to one or more intermediate states, which indicate a potential that the position of the distal end relative to the blood vessel is currently changing or has recently been changed. As explained above, the intermediate states may comprise a tentative-exit state, which indicates a possibility that the distal end is moving, or has just moved, from the inside of the blood vessel to the outside of the blood vessel. Additionally or alternatively, the intermediate states may comprise a tentative-entry state, which indicates a possibility that the distal end is moving, or has just moved, from the outside of the blood vessel to the inside of the blood vessel. Accordingly, an intermediate state indicates a potential of a transition from an origin-state to a destination-state, e.g. for the in-vessel state to the out-of-vessel state (tentative-exit) or *vice versa* (tentative-entry). It is appreciated that an intermediate state is an uncertain transition state, i.e., it indicates that it is possible that a transition has occurred (or is occurring), yet it is also possible that no transition has occurred (or is occurring), and the tendency is due to some other causes (e.g. the patient is moving his hand or is changing position). Therefore, "potential transition" refers herein to an indication that it is possible that a transition has occurred (or is occurring), yet it is also possible that no transition has occurred (or is occurring). The intermediate states are thus temporary states.

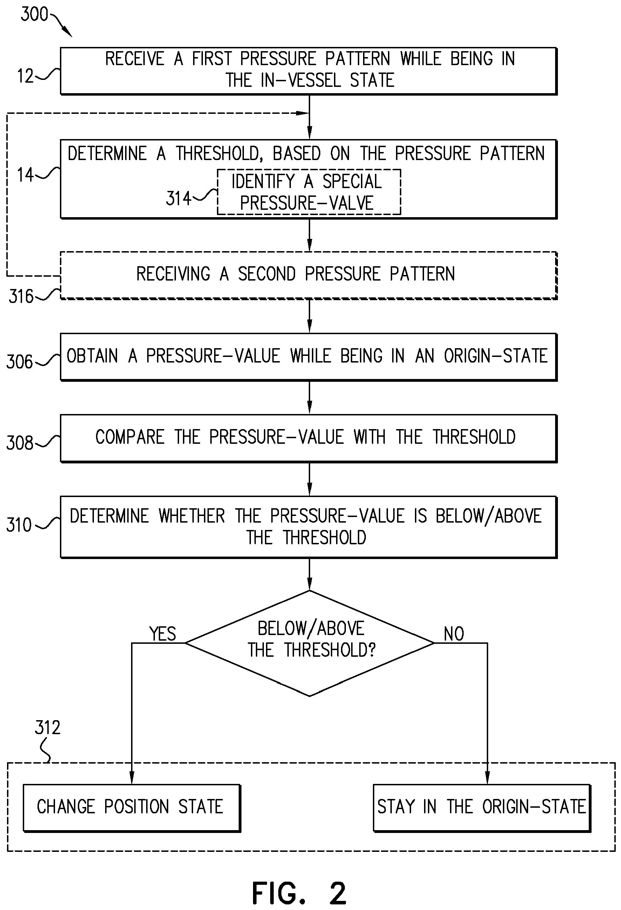

The technique of the present invention also identifies a first initial-entry state being indicative of the first time at which the probe enters the blood vessel, based for example on a calculated pressure threshold. The initial entry state may be defined as the first transition to in-vessel state after zero pressure of a calibrated zero state. Reference is made to **Fig. 2** schematically illustrating, by way of a flow chart, the method **300** for indicating a position of a distal end of a probe relative to a blood vessel of a patient based on the adaptive threshold according to some embodiments of the present invention. In some embodiments, the method **300** further comprises the obtaining of at least one pressure-value being indicative of a certain position state (i.e. origin state) in **306,** comparing the at least one pressure value to the adaptive threshold in **308;** determining whether the pressure-value is below/above the threshold in **310** and indicating a position state of a distal end of a probe relative to a blood vessel in **312.** Indicating a position state of a distal end of a probe relative to a blood vessel in **312** may include indicating that the user should move into the destination-state, or otherwise stay at the origin-state (if the origin-state is the in-vessel state, the destination-state is the out-of-vessel state, and *vice versa).* If the distal end of the probe is in the first position state, and the pressure value is above the adaptive threshold, the position state corresponds to the first position state. If the distal end of the probe is in the first position state and the pressure value is below the adaptive threshold, the position state corresponds to the second position state. If the distal end of the probe is in the second position state, and the pressure value is above the adaptive threshold, the position state corresponds to the first position state. If the distal end of the probe is in the second position state, and if the pressure value is below the adaptive threshold, the position state corresponds to the second position state. The adaptive threshold may be an exit threshold or a re-entry threshold to indicate that the probe is back in the blood vessel, or that it was out of the blood vessel. In a specific and non-limiting example, the first pressure pattern includes a sequence (continuous or not) of 15 samples of pressure measures while the distal end of the probe is located inside the blood vessel. The processing may comprise calculating a moving average of the first pressure pattern, giving a higher weight to the last 5 samples, for example. The average may be for example 30 mbar. The adaptive threshold is then defined to be 24 mbar. More specifically the exit threshold may be defined as 24 mbar and the re-entry threshold as 24 + 3, i.e. 27 mbar. A pressure value is then obtained (i.e. received or measured), while in-vessel state. If the pressure value is 20 mbar, the state is out-of-vessel. If the pressure value is 25, the state is in-vessel state. Additionally or alternatively, the pressure may be obtained, while out-of-vessel state. In this case, if pressure is 20 mbar, for example, the state is out-of-vessel. if the pressure is 28 mbar, the state is in-vessel state.

If the threshold is the exit threshold, the certain position state of **306** is the in-vessel state, and if the threshold is the re-entry threshold, the certain position state is the out-of-vessel state. Moreover, if the threshold is the exit threshold, determining whether the pressure-value is below/above the threshold in **310** comprises determining whether the pressure-value is below the threshold, and if the threshold is the re-entry threshold, determining whether the pressure-value is above the threshold. The exit threshold and/or the re-entry threshold may be determined based on a plurality of pressure-values utilizing one or more of the techniques explained below, and/or any other suitable technique.

In an embodiment, the adaptive threshold may be determined by identifying in the first pressure pattern at least one special pressure value in **314,** and defining the at least one adaptive threshold to correspond to the at least one special pressure value, or to a function of a plurality of special pressure values. For example, the adaptive threshold may be determined based on a statistic derived from the first plurality of pressure-values defining a certain group of pressure-values. The method may comprise determining a subgroup of the group of pressure-values, and determining the statistic based on the subgroup. The subgroup may be the subgroup of the N largest elements out of the M elements of the group of pressure-values (N ≤ M), or any other suitable subgroup. The statistic may be the lowest element within the subgroup, the arithmetic mean of the subgroup, the geometric mean thereof, or any other suitable statistic. The adaptive threshold may be set to the statistic multiplied by a factor. In some example, the factor is higher 0.3 and lower than 0.8. In some examples, the factor is higher 0.4 and lower than 0.7. In some examples, the factors utilized for determining the exit threshold and the re-entry threshold are in the range of about 0.50 to 0.60 and about 0.60 to 0.70, respectively.

In an embodiment, the method comprises obtaining first and second groups of pressure-values while being in the in-vessel state and determining an exit threshold and a re-entry threshold based on the first and second groups of pressure-values, respectively. The first and second groups may be different groups. Alternatively, the first and second groups may coincide. The exit threshold and the re-entry threshold may be equal. Alternatively, the re-entry threshold may be higher than the exit threshold.

In an embodiment, the method may further comprise obtaining new pressure-values in **316** after the threshold has been initially determined, based on an initial group of pressure values, and updating the threshold, based on the new pressure-values. The adaptive thresholds may then define a monotonically increasing function over time (i.e. the updated adaptive threshold will be higher than the precedent adaptive thresholds). The new pressure-values, as well as the elements of the initial group of pressure-values, are obtained while being in the in-vessel state. The new threshold may be determined based on new pressure-values by themselves, based on both the new pressure-values and the initial group of pressure-values, and/or based on current value of the threshold and on the new pressure-values. For example, if the measured pressure at a time T was 60 mbar and the adaptive threshold was calculated to be 30 mbar, then if the measured pressure at a time T+T₁ is 40 mbar, the adaptive threshold remains at 30 mbar. Alternatively, if the measured pressure at a time T+T₁ is 80 mbar, the adaptive threshold may be set to be 40 mbar. In a specific and non-limiting example, a moving group of 10 samples is selected and the global maximum of the moving group is identified and stored. The adaptive threshold may be determined as a certain percentage of the global maximum, and the re-entry threshold may be determined as a certain percentage of the global maximum + a certain constant. The certain percentage of the global maximum may be in the range of about 0.8-0.4 factor. In an embodiment, the method may further comprise obtaining a pressure-value while being in an initial state, comparing the pressure-value with an initial-entry threshold, and determining whether the pressure-value is higher than the initial-entry threshold. The initial-entry threshold may be predetermined. In this connection, it should be noted that determination of the initial-entry threshold, followed by determination of an updated-adaptive-threshold, enable to provide further adaptive thresholds based on enough long-time data. In this way, the invention enables to control that no actual pressure is omitted, if determination of the adaptive threshold starts at the first initial entry. Since the initial-entry is determined when the pressure pattern starts at T₀ of vessel entry, a full history of the pressures is obtained, and larger boundaries are assumed. Therefore, the technique of the present invention enables much less false positive alarms (i.e. out-of-vessel indication when they do not occur). It should be understood that false positive alarms may encourage the practitioner to keep pushing or pulling the probe, although the practitioner is in the vessel already, and therefore may exit the vessel mistakenly. After having been identified, the initial-entry state may be defined as the first transition to in-vessel state after zero pressure of a calibrated zero state.

Additionally or alternatively, the initial-entry threshold may be determined at the beginning of the procedure, based on certain parameters of the patient, such as age, weight, physical condition, body position, or any other suitable parameter.

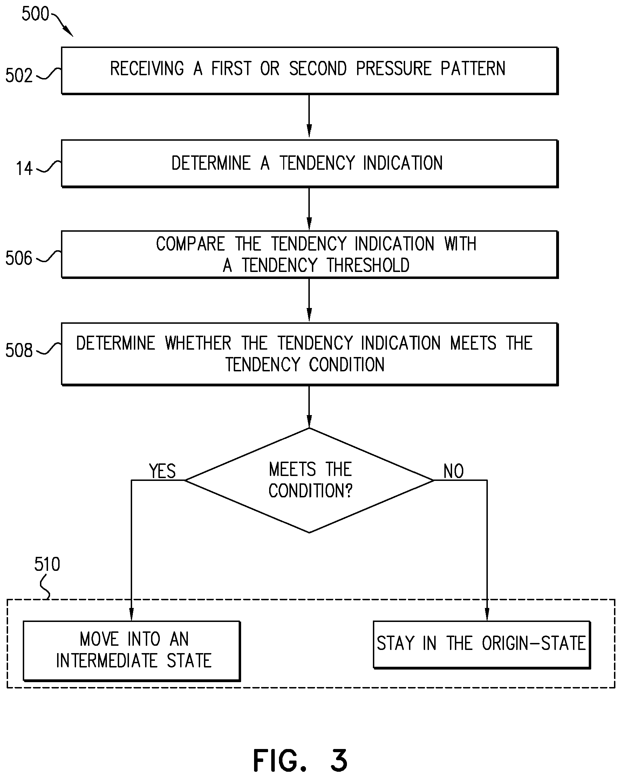

Reference is made to **Fig. 3** schematically illustrating, by way of a flow chart, a method **500** for indicating a position of a distal end of a probe relative to a blood vessel of a patient, utilizing tendencies of pressure-values according to some embodiments of the present invention. The method comprises at least the following: receiving in **502** a first or a second pressure pattern while being in the first position state (i.e. the in-vessel state) or the second position state (i.e. at least partially out-of-vessel state) respectively. In **14,** determining, based on the pressure pattern, a tendency indication being indicative of a certain trend of the pressure pattern over time. If the probe is in in-vessel state, the tendency indication is indicative of a tendency of at least a certain part of the pressure pattern to decrease over time. If the probe is in the out-of-vessel state, the tendency indication is indicative of a tendency of at least a certain part of the pressure pattern to increase over time. The method comprises obtaining or determining a tendency condition, obtaining a certain pressure tendency indication over time of a certain position state and comparing the tendency indication with a tendency condition in **506.** If the origin-state is the in-vessel state, the tendency condition is a tentative-exit condition. If the origin-state is the out-of-vessel state, the tendency condition is a tentative-entry condition. In a specific and non-limiting example, the tendency condition may be 6 millibars per second. If the pressure drops faster than this, i.e. 8 millibars per second, then the condition is met. The tendency indication determines in real-time that currently the pressure drops in 8 millibars per second.

In another specific and non-limiting example, the tendency condition may be a slope of 5 mbar per second. The tendency indication may indicate that now the pressure is dropping fast, e.g. in 7s mbar per second. If the probed is in in-vessel state, since 7 mbar per second is higher than 5 mbar per second, the state is determined to be an intermediate state of a tentative exit state.

The method comprises determining whether the tendency indication meets the tendency condition in **508** and indicating a position state of a distal end of a probe relative to a blood vessel in **510.** If the tendency indication meets the tendency condition, then indicating a position state of a distal end of a probe relative to a blood vessel may include indication that the user should hold and wait to receive a next indication, and whether a change in state is occurring, or if no transition of state is determined. In an embodiment, the tendency indication is a value referred to as "a tendency indication value", and the tendency condition is a threshold referred to as "a tendency threshold". Accordingly, comparing the tendency indication comprises comparing the tendency indication value with the tendency threshold, and determining whether the tendency indication value is higher than the tendency threshold.

In an embodiment, determining the tendency indication value comprises calculating a slope between a pair of elements within the group of pressure-values, and determining the tendency indication value based on the slope.

In an embodiment, determining the tendency indication value comprises fitting a straight line to a subgroup of the group pressure-values, and determining the tendency indication value based on the slope of the straight line.

In an embodiment, the method further comprises obtaining a pressure-value while being in the intermediate state, comparing the pressure-value with a respective threshold, and determining accordingly whether to proceed to the destination-state, or to remain at the intermediate state. For example, when the intermediate state is the tentative-exit state, the method comprises determining whether the pressure-value is lower than the exit-threshold; and when the intermediate state is the tentative-entry state, the method comprises determining whether the pressure-value is higher than the entry-threshold.

In an embodiment, the method further comprises obtaining a new group of pressure-values during a transition time-window, calculating a new tendency indication based on the new group of pressure-values. If the origin state is the in-vessel state, the second tendency indication is indicative of a tendency of the elements of the new group of pressure-values to decrease over time, and if the origin state is the out-of-vessel state, the second tendency indication is indicative of a tendency that elements of the new group of pressure-values increases over time. The method further comprises comparing the new tendency indication with a second tendency condition and determining whether the new tendency indication meets the second tendency condition. If the new tendency meets the second tendency condition, the transition time-window is restarted, otherwise it is moved back to its original state, i.e. to in-vessel state if the tendency was tentative-exit, or to out-of-vessel state, if the tendency was tentative-entry.

In some cases, the probe may comprise an intravascular needle. In some cases, the probe may comprise an intravascular catheter. Additionally or alternatively, the probe may comprise any other suitable device configured to be inserted into the blood vessel of a patient. In some cases, the intravascular catheter may comprise a needle and a cannula. In some cases, the cannula may surround the outer surface of the needle. In some cases, the cannula may reside inside the lumen of the needle. The needle is utilized for penetration into the blood vessel, and for facilitating insertion of the cannula into the blood vessel, and the cannula is inserted into the blood vessel during and/or after penetration of the needle into the blood vessel. Following the entrance of the cannula into the blood vessel, the needle is withdrawn from the blood vessel, leaving the cannula inside the blood vessel. It should be understood that practitioners may assume that a tip of a cannula is in--vessel, after withdrawal of the needle from the cannula, due to a past indication of being in-vessel they received, such as blood in the flashback window of the catheter, or due to some in-vessel indication of some vessel indication device. Such a past indication may be misleading, because until the withdrawal of the needle and during the withdrawal of the needle from the cannula, certain mistaken movements of the practitioner or of the patient, or other physiological causes, may shift the tip of the catheter or the needle or the cannula, in a way that the tip of the cannula is no longer in the vessel, although the practitioner assumes it is, and completes an IV procedure based on that assumption, which may lead to infusion therapy out of the vessels or other incidents. This may be prevented if an indication of the cannula being in the vessel, after starting the needle withdrawal sub process, is provided to the practitioner. Once the practitioner assumes that the tip of the catheter (the needle surrounded by a cannula) is well seated in the vessel, he withdraws the needle out of the cannula, while pushing the cannula forward, deeper into the vessel, leaving the tip of the cannula inside the vessel, and placing and securing the cannula for later infusion therapy, or blood sampling etc. It is appreciated that while withdrawing the needle from the cannula, during the above described process, a pressure drop is generated, which may also be indicated by a vessel indication device as an out-of-vessel state. This indication may also be misleading as to the real location of the tip of the cannula, and the practitioner might be uncertain as to the current tip of cannula location. For this reason, the technique of the present invention enables to provide the force and direction of the needle in relation to the cannula continuously and in parallel to pressure drop. If all conditions occur: drop in pressure, a measured force being higher than a determined force, and a negative direction of the force, then it may be concluded that the drop in pressure reflects a transition to a provisional withdrawal state.

It is further appreciated that when the needle is withdrawn relative to the cannula, while the distal end of the cannula is located within the blood vessel, the pressure decreases temporarily, because the effective volume of the catheter increases, and then increases back, due to fluid-coupling between the blood vessel and a sensing module, through the catheter. Due to the dynamic pressure characteristic of the blood in the vessel, the pressure may increase back to a vessel-pressure levels, and the indication device may indicate that the practitioner should return to the in-vessel state.

Further, the provisional-withdrawal indication, followed shortly by the in-vessel indication, may indicate that the tip of the cannula is in-vessel, after withdrawal of the needle, which in turn, indicates a completion of a procedure. This benefits the practitioner in acknowledging that the cannula is very likely to be in-vessel, and the process has been successful. This further enables a differentiation of the withdrawal-completion from another in-vessel state, providing more certainty to the practitioner that the tip of the cannula has remained in the vessel, which is the objective of the IV procedure.

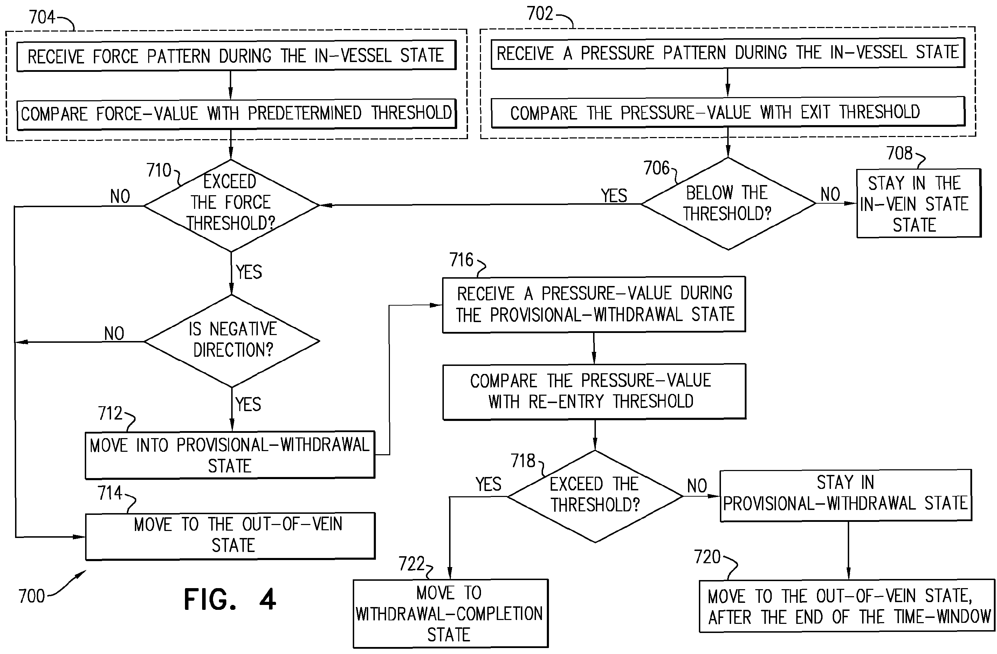

Reference is made to **Fig. 4** schematically illustrating by way of a flow chart a method **700** for indicating a position of an intravascular catheter comprising a needle and a cannula mounted over the needle. The method **700** may comprise at least the following: receiving a first pressure pattern while being in the in-vessel state in **702;** determining an exit threshold; comparing the first pressure-value with a exit threshold; and determining in **706** whether the pressure-value is below the exit threshold. In parallel, the method **700** may comprise at least the following: receiving a force pattern while being in the in-vessel state in **704;** comparing the first force-value with a predetermined threshold; and determining in **710** whether the force-value is below the predetermined threshold. The force pattern comprises a force and a direction measured by an accelerometer in correlation with the pressure drop indication. If the first force-value is above the predetermined threshold, and the direction is negative, and if the first-pressure-value is below the exit threshold, then indicating, in **712,** that the user may move into a provisional-withdrawal state. If the first force-value is above the predetermined threshold, and the direction is positive, and if the first-pressure-value is below the exit threshold, indicating in **714** that the user may move into the out-of-vessel state. If the first force-value is below the predetermined threshold, and if the first pressure-value is below the exit threshold, indicating in **714** that the user may move into the out-of-vessel state. If the first pressure-value is above the exit threshold, then indicating, in 708, that the user may stay in the in-vessel state.

Then the method may comprise receiving a second pressure-pattern while being in the provisional-withdrawal state in **716,** determining a re-entry threshold; obtaining a second pressure-value with a re-entry threshold, and comparing a second pressure-value with a re-entry threshold; and determining whether the second pressure-value exceeds the re-entry threshold in **718.** If the second pressure-value exceeds the re-entry threshold, then indicating in **722** that the user may move to the withdrawal completion state, or otherwise, stay in the provisional-withdrawal state. The method may also comprise identifying residing in the provisional-withdrawal state during a time-window longer than a withdrawal-time threshold, and indicating in **720** that the user can move to the out-of-vessel state after the end of the time-window. In this connection, it should be noted that the withdrawal-time threshold is defined as being an approximative time period being sufficient to enable withdrawal of the needle out of the cannula as measured in real IV procedures, until the time required for the blood pressure to be re-established back to the original vessel pressure. It can be defined to be in the range of about 1.5 to 3 seconds.

In an embodiment, the method may further comprise: obtaining a group of pressure-values while being in the in-vessel state; and determining, based on the group of pressure-values, at least one of the exit threshold and the re-entry threshold.

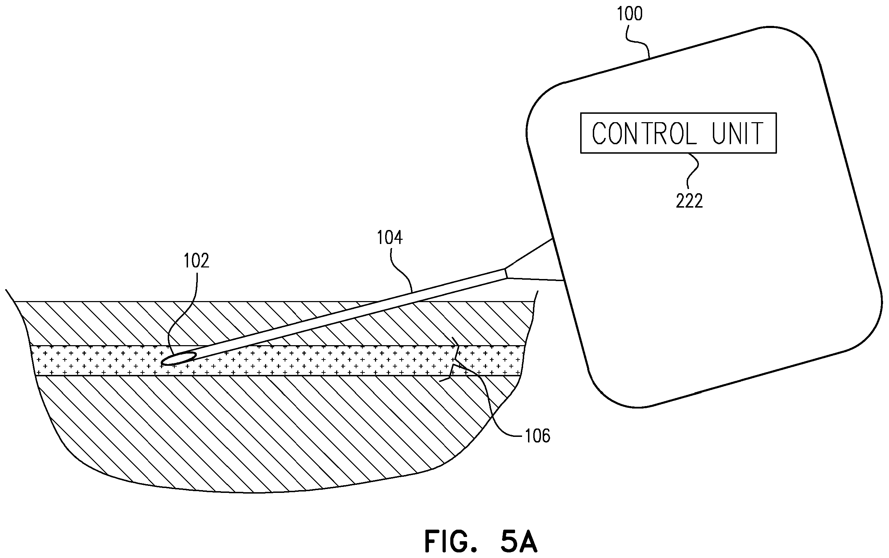

Reference is made to **Fig. 5A** schematically illustrating, by means of a block diagram, a device **100** for determining a position of a distal end **102** of a probe **104** relative to a blood vessel **106** of a patient. The patient may be a human being or an animal, whose body comprises blood vessels. The device **100** is configured to be used with a probe **104** and in a specific and non-limiting example, the device **100** may be coupled to the probe **104** by a luer taper, e.g., by a male luer connector which is configured to be coupled to a female luer connection of the probe. Additionally or alternatively, the device **100** may be coupled to the probe by any other fixed or detachable coupling mechanism suitable for the case. Device **100** comprises a control unit **222** being configured and operable to receive a first pressure pattern comprising a plurality of pressure values being indicative of a pressure of a fluid when the distal end of the probe is located inside the blood vessel; processing the first pressure pattern and determining at least one adaptive threshold being indicative of a transition from a first position state to a second position state or *vice versa* and/or a tendency indication being indicative of a certain trend of the pressure pattern. The first position state defines an in-vessel condition in which the distal end of the probe is located inside the blood vessel and the second position state defines at least one exit condition in which the distal end of the probe is located at least partially outside the blood vessel. The control unit **222** may be implemented by one or more processors (e.g. one or more microprocessors and/or one or more DSPs) which execute suitable software modules, and/or or by dedicated circuitry (e.g. one or more ASICs and/or more FPGAs), or by other suitable devices. The threshold may be an exit threshold, which is utilized to estimate whether the distal end has exited the blood vessel, or a re-entry threshold, which is utilized to estimate whether the distal end has re-entered the blood vessel.

As explained further below, in an embodiment, the control unit **222** may further update the exit threshold and/or the re-entry threshold, based on new pressure-values collected while in-vessel, after the exit threshold and/or the re-entry threshold have been initially determined. Accordingly, the pressure-values utilized for initial determination of the threshold may be referred to herein as an initial group of values.

In some embodiments, the control unit **222** receives a pressure-value while the state indication is at a first state, compares the pressure-value with a threshold, and determines whether to set the state indication to a second state, or to leave it in the first state. The first state and the second state may be referred to also as the origin-state and the destination-state, respectively. In an example, the origin-state is the in-vessel state, the threshold is an exit threshold, and the destination-state is the out-of-vessel state. In this example, the control unit **222** determines whether the value is below the exit threshold. If the value is below the exit threshold, the control unit **222** sets the state indication to the out-of-vessel state. Otherwise, state indication is left in the in-vessel state. In another example, the origin-state is the out-of-vessel state, the threshold is a re-entry threshold, and the destination-state is the in-vessel state. In this example, the control unit **222** determines whether the pressure-value is above the re-entry threshold. If the value is above the re-entry threshold, the control unit **222** sets the state indication to the in-vessel state. Otherwise, the state indication is left at the out-of-vessel state.

In an example, the control unit **222** determines both an exit threshold and a re-entry threshold, as explained above, and the control unit **222** utilizes both thresholds in determining the state indication, as explained above.

The control unit **222** may determine the exit threshold and/or the re-entry threshold based on a plurality of pressure-values utilizing one or more of the techniques explained below, and/or any other suitable technique. In an embodiment, the control unit **222** may determine the exit threshold and/or the re-entry threshold based on a statistic derived from the group of pressure-values. The control unit **222** may determine at least one special pressure value defining a subgroup of the group of pressure-values, and determine the statistic based on the subgroup. Additionally or alternatively, the threshold may be determined by any other suitable function of the statistic.

In an embodiment, the control unit **222** may further update the exit threshold and/or the re-entry threshold, based on new pressure-values obtained from the sensing module after the exit threshold and/or the re-entry threshold were initially determined based on the initial group of values. The new pressure-values, like the initial group of values, are obtained while the state indication is at the in-vessel state. In some cases, the new threshold may be determined based on new pressure-values by themselves. In some cases, the new threshold may be determined based on both the new pressure-values and the initial group of pressure-values. For example, in some cases, the control unit **222** may determine an updated group of pressure-values based on the initial group of pressure-values and the new pressure-values, and update the threshold based on the updated group of pressure-values. In some cases, the new threshold may be determined based on the current value of the threshold (prior to the update) and on the new pressure-values. For example, in some cases, the control unit **222** may determine a provisional value for the exit threshold and/or the re-entry threshold, based on the new pressure-values, utilizing one or more of the techniques explained above, and/or any other suitable technique. Having determined the provisional value, the control unit **222** may determine the updated value of the threshold based on the current value and the provisional value (e.g., by taking the maximum of the two values, or the average thereof, or any other function thereof which is suitable for the case). Additionally or alternatively, the updated value of the threshold may be determined based on any suitable combination of the new pressure-values, the initial group of pressure-values, and/or the current value of the threshold, utilizing any method suitable for the case.

In an embodiment, the state indication is set, at the beginning of the procedure, to an initial state, which indicates that the distal end has likely not entered the blood vessel since the beginning of the procedure. The control unit **222** receives, while the state indication is at an initial state, a pressure-value, compares the pressure-value with an initial-entry threshold, and determines whether the pressure-value is higher than the initial-entry threshold. If the pressure-value is higher than the initial-entry threshold, the control unit **222** sets the state indication to the in-vessel state. Otherwise, the state indication is left to be at the initial state. The initial-entry threshold may be predetermined. Additionally or alternatively, the control unit **222** may determine the initial-entry threshold at the beginning of the procedure, based on certain parameters of the patient, such as age, weight, physical condition, body position, or any other suitable parameter using human-interface (e.g. a button, a touch screen, or other suitable human interface elements) to select a predetermined initial-entry threshold.

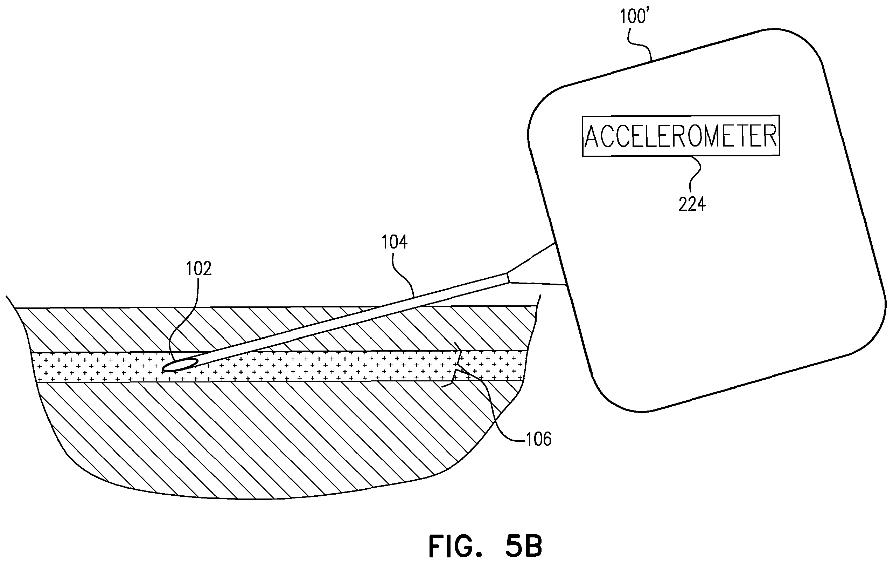

Reference is made to **Fig. 5B** schematically illustrating, by means of a block diagram, a device **100'** for determining a position of a distal end **102** of a probe **104** relative to a blood vessel **106** of a patient. Device **100'** comprises an accelerometer **224** being configured and operable to measure a direction of a movement from a first position state to a second position state or *vice versa* and the transition force from a first position state to a second position state or vice versa and to generate a direction signal being indicative of the transition direction. Accelerometer **224** may be connected to the control unit of the present invention or may be connected to another control unit or another device being configured and operable to receive the signal from the accelerometer **224** and generate a signal being indicative of the position of a probe relative to a blood vessel.

In some embodiments, the accelerometer force and direction may be used in correlation with a pressure drop indication being obtained by the technique of the present invention as described above or by another technique.

In some embodiments, the accelerometer **224** is configured and operable to measure the force/acceleration and the direction of a needle relative to a cannula by using the technique of the present invention or any other technique being aimed at differentiating between the different positions.

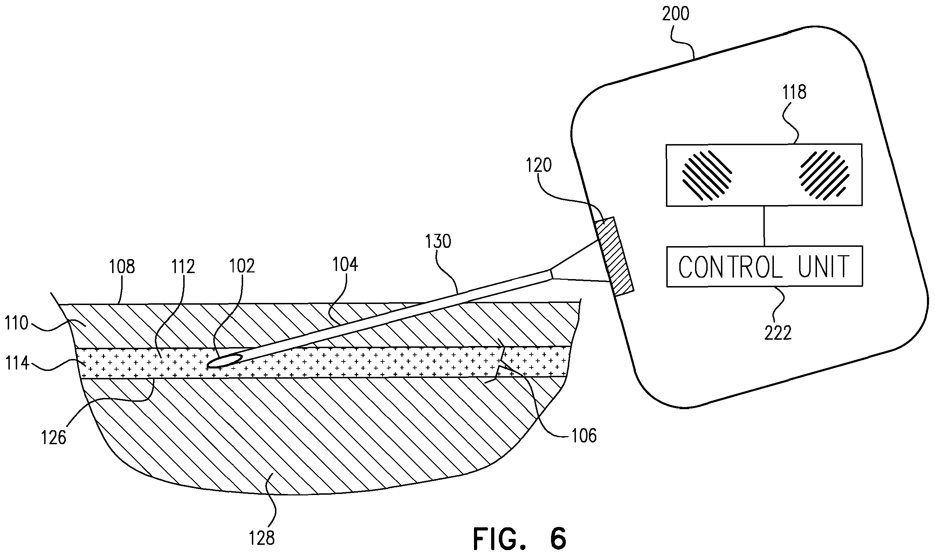

In some embodiments, the device **100'** comprises a pressure sensing module (as illustrated for example in **Fig. 6** below), which produces pressure-values correlating to the pressure of the fluid at the distal end of the probe **104.** The sensing module may comprise a pressure sensor, which is configured to produce pressure-measurements correlating to a pressure applied on the pressure sensor. The pressure sensor may comprise a piezoelectric pressure sensor, a strain gauge pressure sensor, a capacitive pressure sensor, and/or any other suitable pressure sensor. The pressure-values produced by the sensing module may comprise the pressure-measurements produced by the pressure sensor, without further processing. Additionally or alternatively, the sensing module may further comprise a pre-processor, which produces pressure-values by processing the pressure-measurements. For example, the pre-processor may produce the pressure-values by applying a filter to the pressure-measurements. The filter may be a simple moving average, a weighted moving average, a second-order exponential smoothing filter, or any other suitable filter. Additionally or alternatively, the pre-processor may derive pressure-values by any other suitable processing of the pressure-measurements. In some embodiments, the sensing module may comprise an analog-to-digital convertor configured to convert the pressure-measurements into digital values, and the pre-processor may be configured to process the digital values. The pre-processor may be implemented by one or more processors (e.g. one or more microprocessors and/or one or more DSPs) which execute one or more suitable software modules, and/or by a dedicated circuitry (e.g. one or more ASICs and/or one or more FPGAs), or by any other suitable device.

Reference is made to **Fig. 6** schematically illustrating, by means of a block diagram, a device **200** for indicating a position of a distal end **102** of a probe **104** relative to a blood vessel **106** of a patient according to some embodiments of the present invention. In a medical procedure, a physician may maneuver the probe **104** in order to place the distal end **102** inside the blood vessel **106** of the patient, in order to facilitate extraction of blood out of the blood vessel, or delivery of a substance into the blood vessel, or for any other procedure. The physician may push the probe, so that the distal end penetrates the skin **108** of the patient, advances through one or more tissues **110,** penetrates the wall **112** of the blood vessel, and reaches the lumen **114** of the blood vessel. The first entrance of the distal end into the blood vessel may be referred to herein as a first position state or "initial-entry". When the distal end is inside the blood vessel, as illustrated in the figure, the physician may intentionally or unintentionally pull back the probe, and the distal end may exit the blood vessel (e.g. return to the tissue **110**), which is referred to herein as a second position state or "exit". Following an exit, the physician may push the probe again, and the distal end may reach the lumen **114** again, which is referred to as a "re-entry". Exit may also be caused by a "double-puncture", e.g. when the distal end **102** penetrates the opposite wall **126** at the opposite side of the lumen **114** and enters the opposite tissue **128** beyond the opposite wall. After an exit caused by a double puncture, a re-entry may be achieved by pulling back the probe so that the distal end returns into the lumen **114.**

In some embodiments, the device **200** comprises a notification module **118,** being configured and operable to provide to a user a signal being indicative of the position state of the distal end **102** of the probe **104** relative to the blood vessel **106.** The signal may be an audio and/or a visual signal. If an intermediate state is identified (as described above), the signal may be in the form of an alarm, in particular for the states of tentative exit/re-entry. Notification module **118** may be integrated within device **200** as shown in **Fig. 6****.** However, this configuration is not limiting. Additionally or alternatively, the device **100** of **Fig. 5A** may be coupled to an external presentation device, which presents, based on the state indication, a state-signal indicative of the position of the distal end of a probe relative to the blood vessel. The external presentation device may be a dedicated presentation device. Additionally or alternatively, the external presentation device may be a general-purpose device, such as personal computer or a smartphone, which is adapted to present the state-signal (e.g., by executing an appropriate application). Notification module 118 generates a signal being indicative of an in-vessel state or an out-of-vessel state. The in-vessel state indicates that the distal end is likely to be located inside the blood vessel (e.g. within lumen 114), and the out-of-vessel state indicates that the distal end is likely to be located outside the blood vessel (e.g. within the tissue 110,the opposite tissue 128, or outside the body of the patient). The notification module 118, presents, based on the state indication, a signal indicative of the position of the distal end of a probe relative to the blood vessel. The signal may comprise an in-vessel signal, which is presented when the state indication is at the in-vessel state, and an out-of-vessel signal, which is presented when the state indication is at the out-of-vessel state. In an embodiment, when the state indication is at the initial state, the presentation device presents an initial signal. In another embodiment, the notification module **118** may present the out-of-vessel signal, or present no signal, during the initial state. The notification module **118** may comprise a visual presentation device, which presents a visual signal. For example, the notification module **118** may emit light of a certain color (e.g., yellow) when the state indication is at the out-of-vessel state, and a different color (e.g., green) when the state indication at the in-vessel state. Additionally or alternatively, the notification module **118** may comprise an audio presentation device, which presents an audio signal. For example, the notification module **118** may produce an audio signal at a certain frequency when the state indication at the out-of-vessel state, and produce an audio signal at a different frequency, when the state indication is at the in-vessel state. Additionally or alternatively, the notification module **118** may comprise a vibration signaling device. Such examples of signals are not limiting, and other signals, for example vibration signals, and/or any other signals, or combinations of signals, may also be utilized.

The device **200** maintains the state indication based on pressure-values correlating to the pressure of the fluid at the distal end **102** of the probe **104** (e.g., the pressure-values are higher when the pressure at the distal end is higher). When the distal end is located inside the blood vessel **106,** the pressure-values correlate to the pressure within the blood vessel **106,** e.g. the pressure of the blood within the lumen **114.** When the distal end is located outside the blood vessel, the pressure-values correlate to the pressure outside the blood vessel, e.g. the pressure of the tissue or interstitial fluid in tissue **110** or opposite tissue **128.** In some embodiments, based on the assumption that the pressure inside the blood vessel is higher than the pressure outside the blood vessel, the device **200** estimates the likelihood of the distal end to be located inside or outside the blood vessel by comparing the pressure-values with one or more thresholds, and setting the state indication accordingly (e.g., sets the state indication to the in-vessel state when the pressure-values exceed a certain threshold, and/or sets the state indication to the out-of-vessel state when the pressure-values are below a certain threshold). The one or more thresholds are determined adaptively, based on pressure-values correlating to the actual pressure within the blood vessel, i.e. pressure-values obtained during an in-vessel state.

As described in the embodiment of **Fig. 5B****,** the device **200** may also comprise a pressure sensing module **120,** which produces pressure-values correlating to the pressure of the fluid at the distal end of the probe **104.** In some cases, sensing module **120** may be fluidly coupled to the lumen **130** of the probe (e.g., by being fluidly coupled the proximal end **130** of the probe). It is noted that the coupling may be fixed and/or detachable. In some cases, for example, sensing module **120** may be fluidly coupled to a female luer connection of device **200,** which is coupleable to a male luer connection of the probe. Since the lumen of the probe is fluidly coupled to its distal end, the pressure measured by the sensing module **120** correlates to the pressure of the fluid at the distal end of the probe. It is appreciated that "fluid" and "fluidly coupled" may refer to gas as well as to liquid. In some cases, for example, the lumen **130** may contain gas (e.g., air), which is fluidly coupled to the distal end of the probe. Since the pressure of the air within the lumen of the probe correlates to the pressure at the distal end of the probe, the pressure-values produced by the sensing module **120** also correlate to the pressure at the distal end of the probe. The pressure at the distal end of the probe may, according to the position of the distal end of the probe, be the pressure of the blood within the blood vessel, the pressure of a tissue other than the blood vessel, or the pressure of the atmosphere (when the probe is outside the body of the patient).

The sensing module **120** may comprise a pressure sensor, which is configured to produce pressure-measurements correlating to a pressure applied on the pressure sensor. The pressure sensor may comprise a piezoelectric pressure sensor, a strain gauge pressure sensor, a capacitive pressure sensor, and/or any other suitable pressure sensor. The pressure-values produced by the sensing module **120** may comprise the pressure-measurements produced by the pressure sensor, without further processing. Additionally or alternatively, the sensing module may further comprise a pre-processor, which produces pressure-values by processing the pressure-measurements. For example, the pre-processor may produce the pressure-values by applying a filter to the pressure-measurements. The filter may be a simple moving average, a weighted moving average, a second-order exponential smoothing filter, or any other suitable filter. Additionally or alternatively, the pre-processor may derive pressure-values by any other suitable processing of the pressure-measurements. In some embodiments, the sensing module **120** may comprise an analog-to-digital convertor configured to convert the pressure-measurements into digital values, and the pre-processor may be configured to process the digital values. The pre-processor may be implemented by one or more processors (e.g. one or more microprocessors and/or one or more DSPs) which execute one or more suitable software modules, and/or by a dedicated circuitry (e.g. one or more ASICs and/or one or more FPGAs), or by any other suitable device.

In an embodiment, the notification module **118** presents an in-vessel signal and an out-of-vessel signal when the state indication is at the in-vessel state and the out-of-vessel state, respectively, and further presents a tentative-exit signal when the state indication is at the tentative-exit state, and/or presents a tentative-entry signal when the state indication is at the tentative-entry state. The signals may be visual and/or audio signals. For example, the in-vessel signal and out-of-vessel signal may be steady lights of a first color (e.g. green) and a second color (e.g. yellow), respectively, while the tentative-exit state and the tentative-entry states may be blinking lights of the second color and the first color, respectively. Additionally or alternatively, the in-vessel signal and out-of-vessel signal may be steady audio signals at a first frequency and at a second frequency, respectively, while the tentative-exit state and the tentative-entry states may be audio signals at the second frequency and the first frequency, respectively, which are subject to on-off modulation. Additionally or alternatively, any other set of visual and/or audio signals may be utilized.

As noted above, the intermediate states are temporary states. In an embodiment, the control unit **222** obtains pressure-values during the intermediate state, compares the pressure-values with a respective threshold, and determines accordingly whether to proceed to the destination-state, or to remain at the intermediate state; and if the state indication remains at the intermediate state for a period longer than a time-out threshold, the control unit **222** returns to the origin-state. For example, if the intermediate state is the tentative-exit state, the control unit **222** determines whether the pressure-value is below an exit-threshold. If the pressure-value is below the threshold, the control unit **222** sets the state indication to the exit state, otherwise it leaves the state indication in the tentative-exit state. Likewise, if the intermediate state is the tentative-entry state, the control unit **222** determines whether the pressure-value is above an entry-threshold. If the pressure-value is below the threshold, the control unit **222** sets the state indication to the in-vessel state, otherwise it leaves the state indication in the tentative-entry state.

If the control unit **222** identifies that the state indication has resided at the intermediate state during a transition time-window longer than a transition time-out threshold, without proceeding to the destination-state, the control unit **222** returns the state indication to the origin-state. However, in an embodiment, the control unit **222** may restart the transition time-window when it obtains an indication that the pressure-values are continuing to change (increase or decrease, as appropriate to the case). The control unit **222** may obtain a second group of pressure-values during the transition time-window and calculate a second tendency indication based on the second group of pressure-values. If the origin-state is the in-vessel state, the second tendency indication is indicative that elements of the second group of pressure-values continue to decrease over time, and if the origin-state is the out-of-vessel state, the second tendency indication is indicative that elements of the second group of pressure-values continue to increase over time. The control unit **222** then compares the second tendency indication with a second tendency condition and determines whether the second tendency indication meets a second tendency condition. If the second tendency indication meets the second tendency condition, the control unit **222** restarts the transition time-window. In some cases, the second tendency condition may be equal to the first tendency condition. For example, the second tendency condition may be a second tendency threshold, which is equal to the first tendency threshold. In other cases, the second tendency condition may be different from the first tendency condition. For example, the second tendency condition may be a second tendency threshold, which is lower than the first tendency threshold.

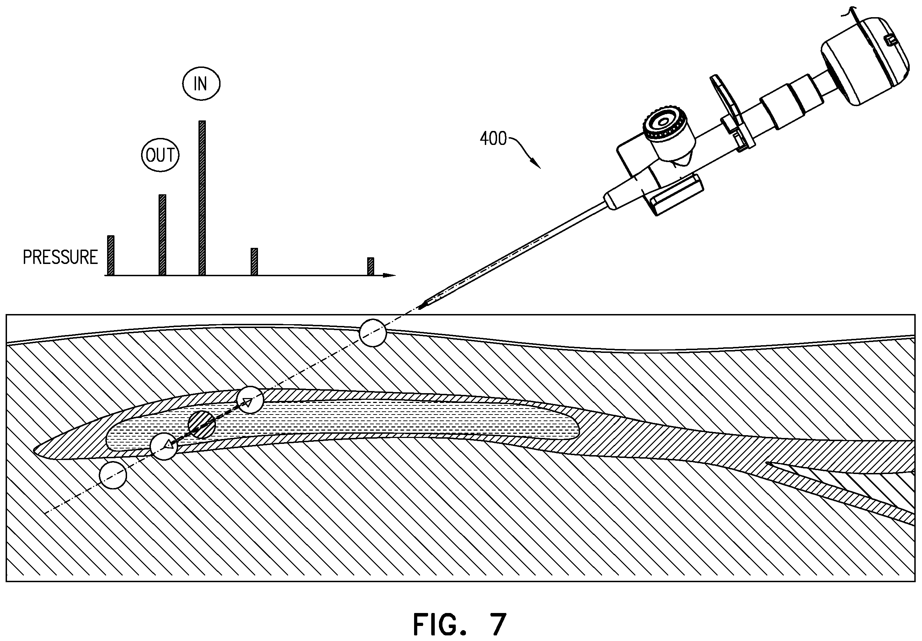

Reference is made to **Fig. 7** schematically illustrating a device **400** for determining a position of a distal end **102** of a probe **104** relative to a blood vessel **106** of a patient according to some embodiments of the present invention.

The state indication is set to an intermediate state when the pressure pattern indicates a tendency of the pressure at the distal end to change in a certain direction (e.g. to increase or to decrease, as appropriate to the case). For example, when the state indication is at the in-vessel state, a tendency of the pressure-values to decrease might indicate that the distal end is during a transition from the inside of the blood vessel to the outside of the blood vessel, and the transition causes the pressure within the probe to start decreasing. Additionally or alternatively, a tendency of the pressure-values to decrease might indicate that the distal end has recently exited the blood vessel, and the pressure within the probe is continuing to decrease. Likewise, when the state indication is at the out-of-vessel state, a tendency of the pressure-values to increase might indicate that the distal end is during a transition from the outside of the blood vessel to the inside of the blood vessel, and/or an indication that the distal end has recently entered the inside of the blood vessel. It is noted, however, that the tendency of change might also be due to other causes, for example a change in position of the body of patient, a change in the arterial blood pressure of the patient, which might affect the venous blood pressure thereof, or another reason applicable to the case.

The intermediate states are temporary states. After a transition from the origin-state (e.g. the in-vessel state or the out-of-vessel state) to an intermediate state (e.g. the tentative-exit state or the tentative-entry state, respectively), control unit **222** may either indicate, based on further pressure-values, to complete the transition to destination-state (e.g. to the out-of-vessel state, or to the in-vessel state), or decide to return to the origin-state. For example, if the origin-state is the in-vessel state and the pressure-values decrease below a certain threshold, the control unit **222** may indicate to move into the out-of-vessel state. Otherwise, if the pressure-values fail to decrease below the certain threshold during a time-window, the control unit **222** returns to the in-vessel state. Likewise, if the origin-state is the out-of-vessel state and the pressure-values increase above a certain threshold, the control unit **222** moves into the in-vessel state. Otherwise, if the pressure-values fail to increase above the threshold during a given period, the control unit **222** returns to the out-of-vessel state.

The control unit **222** may receive a pressure pattern while the state indication is at an origin stable state, which is one of the in-vessel state or the out-of-vessel state. Based on the pattern, the control unit **222** determines a tendency indication. If the origin-state is the in-vessel state, the tendency indication is indicative of a tendency of the elements of the pressure pattern to decrease over time, and if the origin-state is the out-of-vessel state, the tendency indication is indicative of a tendency of the elements of the pressure pattern to increase over time.

The control unit **222** compares the tendency indication with a tendency condition. If the origin-state is the in-vessel state, the tendency condition is an exit-tendency condition, and if the origin-state is the out-of-vessel state, the tendency condition is an entry-tendency condition. The control unit **222** determines whether the tendency indication meets the tendency condition. If the tendency indication meets the condition, the control unit **222** sets the state indication to an intermediate state, which indicates a tentative assumption of a potential transition from the origin-state to the destination-state (if the origin-state is the in-vessel state, the intermediate state is the tentative-exit state, and if the origin-state is the out-of-vessel state, the intermediate state is the tentative-entry a state). Otherwise, the control unit **222** leaves the state indication in the original stable state.

In an embodiment, the tendency indication may be represented by a value, referred to as a "tendency indication value", which is indicative of the tendency of the elements of the group of pressure-values to decrease over time (if the origin-state is the in-vessel state) or to increase over time (if the origin-state is the out-of-vessel state). Accordingly, the tendency condition may be represented by a threshold, referred to as a "tendency threshold", and comparing the tendency indication with the tendency condition may be performed by comparing the tendency indication value with the tendency threshold. In an example, the tendency indicator may indicate a change of the pressure in millibar (decrease or increase, as applicable to the case), and the tendency threshold may be a value higher than 3 millibars and lower than 6 millibars. If the tendency indication exceeds the tendency threshold, the control unit **222** sets the state indication to an intermediate state; otherwise, the control unit **222** leaves the state indication in the original stable state. Additionally or alternatively, the tendency may be indicated, and the tendency condition may be specified, by any other methods suitable for the case.

The control unit **222** may determine the tendency indication value, based on a group of pressure values according to any of the techniques presented in the following, any suitable combination thereof, or any other suitable technique. For example, the control unit **222** may calculate the slope between a pair of elements within the group of pressure-values and determine the tendency indication value based on the slope. The control unit **222** may also fit a geometrical shape such as a straight line to elements of the group of pressure-values (e.g. by regression, or by any other suitable technique), and determine the tendency indication value based on a slope of the fitted straight line. As described above, the continuously received pressure pattern may be processed to identify at least part of the pattern being indicative of relevant pressures that should be considered as a relevant sample in the determination of crossing the threshold or of the tendency condition. The relevant sample may be extracted from the continuously received pattern. In a specific and non-limiting example, the first pressure value of the relevant sample may be the pressure value having the highest value and the last pressure value of the relevant sample may be the pressure value having the lowest value. A difference between the first pressure value and the last pressure of the sample may be then calculated and represented as a millibar per second. This difference, or the slope, is indicative of fast change in the pressures which may be indicative of exiting or entering the vessel, i.e. from relatively high pressure to relatively low pressure, or *vice-versa.*

The control unit **222** may determine the tendency indication value by multiplying the slope by an appropriate factor. It is noted that the meaning of the tendency indication value depends on the state indication. If the origin-state is the in-vessel state, the tendency indicator value is indicative of decrease over time, and if the origin-state is at the out-of-vessel state, the tendency indicator value is indicative of increase over time. Therefore, the factor is negative in the first case, and positive in the second case.

In some cases, the tendency may be indicated by two or more tendency indication values. For example, the tendency indication may comprise two or more values indicative of two or more of the following values: the difference between the first element and the last element of the group of pressure-values, the minimum difference between adjacent elements within the group, the difference between the first element and the highest/lowest element within the group, the difference between the highest/lowest element within the group and the last element and the group, and any other suitable value derived from the elements of the group. In some cases, the tendency condition may be specified by two or more respective tendency thresholds. In some cases, comparing the tendency indication with the tendency condition may comprise comparing the two or more tendency indication values with two or more respective tendency thresholds.

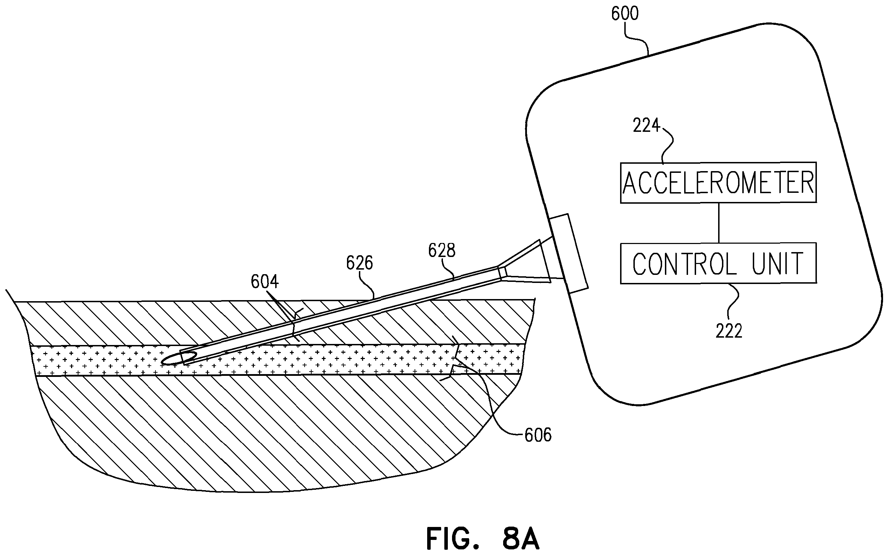

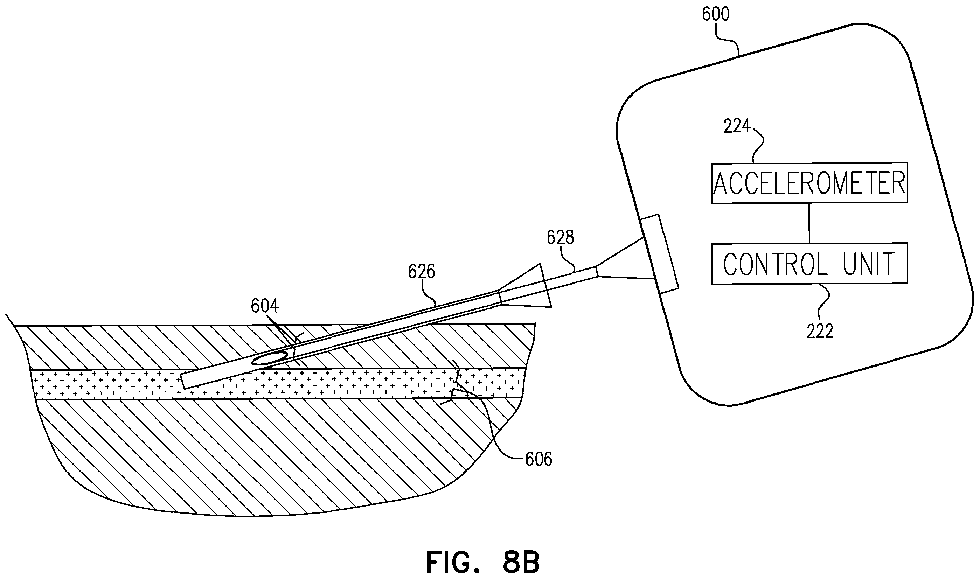

Reference is made to **Fig. 8a** and **Fig. 8b** schematically illustrating an embodiment of a device **600** for indicating a position of an over-the-needle intravascular catheter **604.** The catheter **604** comprises a needle **626,** and a cannula **628** mounted over the needle. Typically, the distal tip of the needle **626** extends further than the cannula tip (e.g. to about 1 mm) to enable the needle tip to puncture the skin and the proximal vessel wall. The needle **626** is accommodated inside the cannula **628** and is tightened to some degree, creating a friction force in the case of pulling out the needle **626** from the cannula **628.** It should be understood that when the practitioner decides that the tip of the needle is well seated within the vessel, he assumes that the tip of the cannula is also within the vessel. However, it may occur that the tip of the needle is not well seated in the vessel (e.g. is partially sighted), in a way that the cannula distal end is actually not completely or not at all within the vessel. Once the practitioner establishes that the tip of the catheter is within the vessel, he should hold the cannula steady, and push it forward into the vessel, while pulling back the needle. This momentarily enlarges the volume, thus momentarily dropping the pressure. At the same time, while retracting the needle from the cannula, a friction force between the needle and the cannula is generated, to resist such pulling action. The present invention enables to identify a relative movement between the cannula and the needle and to detect a pull off of the needle and the transition of the needle along the cannula in a certain direction. This may be implemented by providing device **600** comprising control unit **222** of **Fig. 5A** **or** **Fig. 7** above and an accelerometer **224** being configured and operable to measure a direction of the transition from a first position state to a second position state or *vice versa* and to generate and transmit to control unit **222** a direction signal being indicative of the transition direction.

**Fig. 8a** illustrates a stage where the distal end of the needle is extended further to the distal end of the cannula, thereby facilitating insertion of the catheter into a blood vessel **606** of the patient; and **Fig. 8b****,** illustrates a stage where cannula is further advanced into the blood vessel **606,** and the needle is withdrawn, leaving the distal end of the cannula within the blood vessel **606.** The state indication is determined by a control unit **222,** based on pressure-values correlating to the pressure of the fluid at the distal end of the catheter **604.** It is appreciated that when the distal end of the needle is extended further to the distal end of the cannula, as in **Fig. 8a****,** the distal end of the catheter constitutes the distal end of the needle, and when the distal end of the cannula is extended further to the distal end of the needle, as in **Fig. 8b****,** the distal end of the catheter constitutes the distal end of the cannula. The state indication may be set to the in-vessel state, and to the out-of-vessel state. The state indication may also be set to a provisional-withdrawal state. The provisional-withdrawal state is indicative of two possible alternatives: a first alternative that the needle **626** has been withdrawn relative to the cannula **628,** leaving the cannula within the blood vessel **606,** as illustrated in **Fig. 8b****,** and a second alternative that the distal end of the catheter has exited the blood vessel **606.**

It is appreciated that when the distal end of the catheter exits the blood vessel, the pressure sensed by the sensing module decreases, and remains at a low level as long as the distal end is located outside of the blood vessel. It is further appreciated that when the needle is withdrawn relative to the cannula, while the distal end of the cannula is located within the blood vessel, the pressure decreases temporarily, because the effective volume of the catheter increases, and then once again increases, due to fluid-coupling between the blood vessel and the sensing module, through the catheter, and due to the dynamic pressure characteristic of the blood in the vessel. Accordingly, the state indication is set to the provisional-withdrawal state when the pressure-values decrease below an exit-threshold.

The following cases may occur:
(1) If, while being in the provisional-withdrawal state, the pressure-values once again increase within a certain time-window, and exceed a second re-entry threshold, the state indicator is set back to the in-vessel state, thereby the state indication may indicate that the cannula is located within the blood vessel.
(2)If the pressure-values do not exceed a second re-entry threshold within the certain time-window, the state indication is set to the out-of-vessel state, thereby indicating that the catheter has exited the blood vessel.
(3) It is also possible that after a certain time-window, the catheter is drawn back by the practitioner, and exceeds a second re-entry threshold, and therefore the state indicator is set back to the in-vessel state.

In some embodiments, the present invention enables to differentiate between cases (1) and (3) and to determine whether the provisional withdrawal state, followed by an in-vessel state, indicates an in-vessel state of the needle, or indicates withdrawal of the needle from the cannula. This may be implemented by using the accelerometer force and direction in correlation with the pressure drop indication. The transition direction and force of the needle relative to the cannula in this case may be correlated to the pressure decrease in a time-window.

In a specific and non-limiting example, if the accelerometer measures a force in the range of 2-6 gr, or 1-8 gr, overcoming the friction force between the needle and the cannula, and some momentum force, in the negative direction (i.e. the direction in which the needle direction is opposite to the cannula direction), and a pressure drop below the threshold is also measured at the same time, the control unit **222** determines that the needle has been withdrawn relative to the cannula.

If the above case occurs, the immediately following in-vessel indication, as described, indicates a withdrawal completion and the control unit **222** may provide a signal indication of successful withdrawal completion. This benefits the practitioner in acknowledging that the cannula is very likely to be in the vessel, and that the process has been successful. The accelerometer **224** is thus configured and operable to measure the force/acceleration as measured in grams (e.g. in the range of about 2 and 5 grams) and the direction of the needle relative to the cannula. This feature is clearly different from other motions during the IV insertion procedure, especially after being in an "in-vessel" state.

It should also be noted that before the initial entry state (i.e. first-time "in-vessel" state) occurs, which may include also the time before penetrating the skin at all, the motion or direction of the needle may vary greatly, but the abovementioned process uses the motion and direction only after at least the initial entry state has occurred. However, if an "in-vessel" state is identified, this means that the practitioner is acting on a specific axis, i.e. the needle axis. Then the practitioner can only (1) push the needle forward while puncturing the distal wall of the vessel being measured as positive direction and certain force, (2) withdraw the needle backward being measured as negative direction, which produces a pressure drop, but also a small acceleration force or (3) withdraw the needle from the cannula producing pressure drop as explained above, and being measured as a negative direction, with a certain force due to the resistance of the friction between the needle and the cannula. To differentiate between (2) and (3) above, a certain difference in force measured may be identified, as well as recovery of the pressure within a short time, and transition back to "in-vessel" state. This enables to determine that process (3) is in a withdrawal-completion state, indicating successful insertion of the cannula into the vessel after pulling out the needle.

Otherwise, if the force and direction measured by the accelerometer **224** is a force in the range of above as 1-5 g in the positive direction, while the pressure dropped below an exit-threshold as described, then the control unit **222** determines that the needle exited the vessel to the other side, puncturing the distant wall of the vessel, and the control unit may provide a signal indication of out-of-vessel state.

In an embodiment, the control unit **222** obtains a first pressure-value while the state indication is in the in-vessel state, compares the first pressure-value with a threshold, determines a first determination that the pressure-value is below the threshold, and sets the state indication, based on the first determination, to the provisional-withdrawal state. In some cases, the control unit **222** obtains, while being in the provisional-withdrawal state, a force and direction of the accelerometer **224,** (e.g. produced and stored at the time of the pressure drop in real time), and compares the received force and direction to an adaptive threshold being indicative of a direction and transition force threshold. The transition force threshold may be predetermined. More specifically, a predetermined transition force threshold may be calculated and/or may be adapted/fine-tuned to variance of forces being measured in the procedure itself. For example, it may be based on research identifying the force required to pull a needle from a cannula, based on average forces, average different needles, and on a learning curve being adapted to detect such a change during the needle insertion, and differentiate from the general pattern of forces starting at first entry state, a certain point in time. In a specific and non-limiting example, the transition force threshold may be in the range of about 2-5 grams.

If the control unit **222** determines that the force and direction does not exceed the withdrawal-completion force threshold and direction requirement, the control unit **222** leaves the state indication at the provisional-withdrawal state. Otherwise, if the force and direction exceed the withdrawal-completion force and direction threshold, the control unit **222** generates a state indication indicative of the withdrawal completion state.

In some cases, the control unit **222** identifies that the state indication has been in the provisional-withdrawal state during a time-window equal to or longer than a withdrawal-time threshold, and sets the state indication to the out-of-vessel state, after the end of the time-window, for example, more than 1-2 seconds after having identified the provisional-withdrawal state.

In an embodiment, the control unit **222** compares the tendency indication with the withdrawal tendency condition. If the tendency indication meets the tendency condition, the control unit **222** sets the state indication to the provisional-withdrawal state. Otherwise, if the tendency indication does not meet the tendency condition, the control unit **222** leaves the state indication in the in-vessel state. The withdrawal tendency condition may be specified by and compared to one or more of the methods described above of the tendency condition, and/or by any other suitable methods. Provisional-withdrawal state which was determined following tendency indication which meets the tendency condition, may be followed with a force and negative direction being compared to threshold, and followed by recovery of pressure above re-entry threshold, then being indicated as a withdrawal completion state.

In an embodiment, the state indication may, in some cases, in addition to the in-vessel state, out-of-vessel state, and the provisional-withdrawal state, be set to the tentative-exit state, which is indicative of a potential transition of the distal end of the catheter out of the blood vessel. The notification module may further be configured to present the tentative-exit signal when the state indication is equal to a tentative-exit state. In an embodiment, the control unit **222** compares the tendency indication with two tendency conditions: the withdrawal tendency condition, and a tentative-exit tendency condition. If the tendency indication meets the withdrawal tendency condition, the state indication is set to the provisional-withdrawal state; otherwise, if the tendency indication meets the tentative-exit tendency condition, the state indication is set to the provisional-exit state and otherwise, the state indication is left in the in-vessel state.

It is noted that, in some cases, the tendency indications may be represented by tendency indication values, and the tendency conditions may accordingly be represented by tendency thresholds. In some cases, the tentative-exit tendency threshold may be a value higher than 3 millibars and lower than 6 millibars per second, and the withdrawal tendency threshold may be a value higher than 4, and lower than 8 millibars per second.

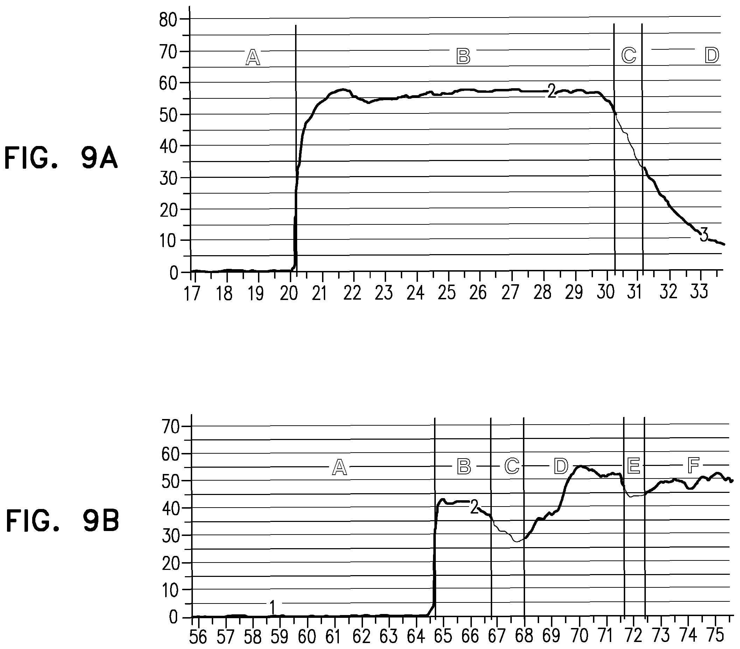

Reference is made to **Figs. 9a-9b** graphically showing two different pressure patterns being processed by using the teachings of the present invention. The technique of the present invention processes the pressure pattern and determines that the pattern of **Fig. 9a** defines four different regions **A, B, C** and **D.** The regions **A** and **D** were defined to be indicative of an out-of-vessel state. Region **B** was defined to be indicative of an in-vessel state. The region **C** shows a trend of decreasing pressure being defined as a tentative-exit state as defined above. The technique determined that the state once again reverted to an "out of-vessel" state.

**Fig. 9b** defines six different regions **A-F.** The region **A** was defined to be indicative of an out-of-vessel state. Regions **B, D** and **F** were defined to be indicative of an in-vessel state. Regions **C** and **E** show a trend of decreasing pressure being defined as a tentative-exit state as defined above. However, since the drop in pressure was brief, the technique determined that the state reverted back to an "in-vessel" state.

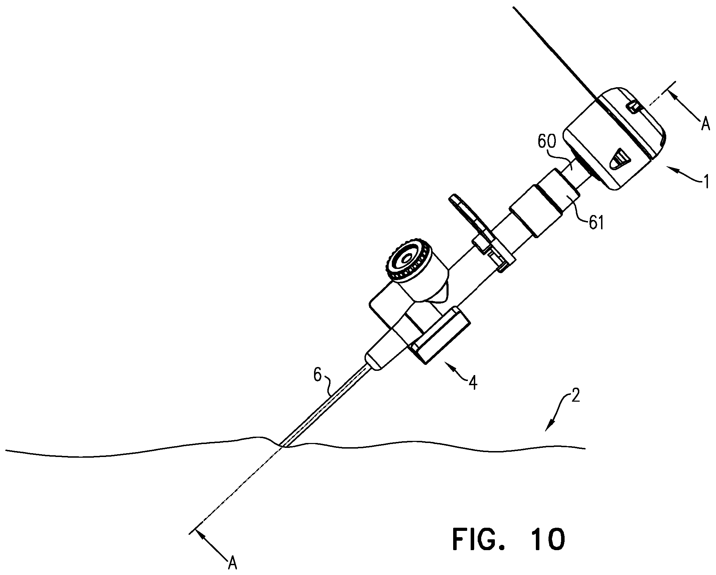

Reference is made to **Figs. 10 to 13****,** illustrating an example of a device **1** with a proximal end of a probe **4** connected thereto, according to an example of the presently disclosed subject matter.

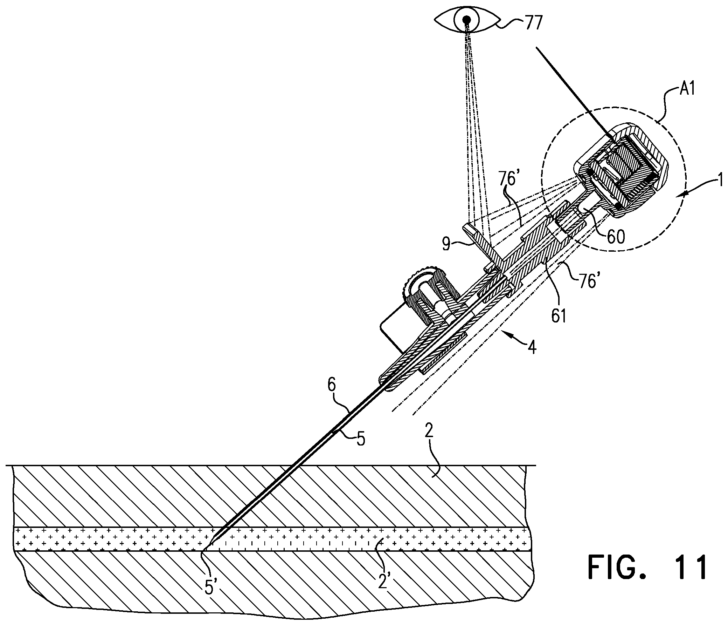

As shown in **Figs. 10** and **11****,** the probe **4** comprises a needle **5** and a cannula **6,** and the needle **5** has passes through a skin **2** of a patient (e.g., a human being or an animal) and is so that distal end **5'** of the needle **5** is positioned in a blood vessel **2'** of the patient. The probe 4 can be any known in the art probe configured to enable flow of fluid into and/or out of the blood vessel, for example flow of blood out of the blood vessel **2'** or flow of substance, such as medication, into the blood vessel **2'.** The cannula **6** surrounds the needle **5** and is configured to be inserted into the blood vessel **2'** together with the needle **5,** and to be left therewithin after the needle **5** is withdrawn from the body of the patient. It should be appreciated, though, that the probe **4** is brought here as a non-limiting example, and other types of probes, for example a probe comprising a cannula having a sharp tip, which facilitates penetrating into the vessel, or any other probe suitable for the case, may be utilized. According to another example, the probe can be a syringe which has a needle without a cannula.

In a vascular access procedure, a practitioner performing the procedure maneuvers the probe **4** in order to place its distal end **5'** inside the blood vessel **2'** of the patient, thereby facilitating extraction of blood out of the blood vessel **2',** delivery of a substance into the vessel, and/or any other suitable operation. The practitioner pushes the probe **4,** so that the distal end **5'** of the needle **5** (and of the probe **4**) penetrates a skin **2** of the patient, advances through one or more tissues, penetrates a wall of the blood vessel **2',** and enters into the blood vessel **2'.** It should be appreciated that when the distal end **5'** of the needle enters the blood vessel **2',** the practitioner may proceed with the procedure. In some cases, for example, the practitioner may advance the cannula 6 relative to needle **5,** withdraw the needle **5** while maintaining the cannula 6 within the blood vessel **2',** extract blood out of blood vessel **2',** deliver substance into the blood vessel **2',** and/or perform any other suitable operation. It should be further appreciated that once the distal end **5'** of the needle enters the blood vessel **2'**, the practitioner should manipulate the probe accordingly. For example, the practitioner should avoid "double-puncture", i.e., situation where the distal end penetrates an opposite wall at an opposite side of the vessel and enters an opposite tissue beyond the opposite wall. Therefore, obtaining indication of the position of the probe **4** relative to the blood vessel **2'** may assist the practitioner in performing the vascular access procedure.

The device **1** is connected to the probe 4 for indicating a position of the distal end **5'** of the needle of the probe relative to the blood vessel **2'.** The device comprises a housing **20** having a housing distal portion **25** with at least partially light-permeable external wall **27** and a housing proximal portion **30.** The housing **20** has a tubular shape.

The housing **20** encapsulates therein a bordering member in the form of a fluid-tight Printer Circuit Board (PCB) **40** positioned substantially perpendicular to a longitudinal axis **L** of the housing **20** and having a first face **42** facing the housing distal portion **25** and an opposite second face **44** facing the housing proximal portion **30.** In other words, the PCB divides the housing **20** to the housing proximal portion **30** and the housing distal portion **25.** The PCB **40** provides mechanical support to components located at the housing proximal portion and constitutes a substrate for electric connectivity to one or more electronic components located in the housing, as detailed below.

The housing distal portion **25** has a fluid chamber **50** formed therein. The fluid chamber **50** encloses a fluid tight volume **52** configured for receiving fluid therein. The fluid tight volume **52** of the fluid chamber **50** is enclosed between the fluid-tight PCB **40,** the light-permeable external wall **27** and an O-ring **28.** The light-permeable external wall **27** is at least partially concave with respect to the fluid chamber **50.**

The housing **20** further has a housing opening **60** disposed at the housing distal portion **25** and configured for establishing fluid communication between the distal end **5'** of the needle **5** and the fluid-tight volume **52** of the fluid chamber **50.** As shown in the drawings, the housing opening **60** is formed as a fitting connected to a respective fitting 61 (e.g., a female Luer-tapper) at a proximal end of the probe **4.** The connection allows the pressure of fluid (e.g. air) gas within the fluid-tight volume **52** to be correlative to the pressure within in the needle **5** of the probe **4.** Moreover, the Therefore, the pressure of air within the fluid-tight volume **52** is substantially equal to the pressure within the needle **5** of the probe **4** and the distal end **5'.**

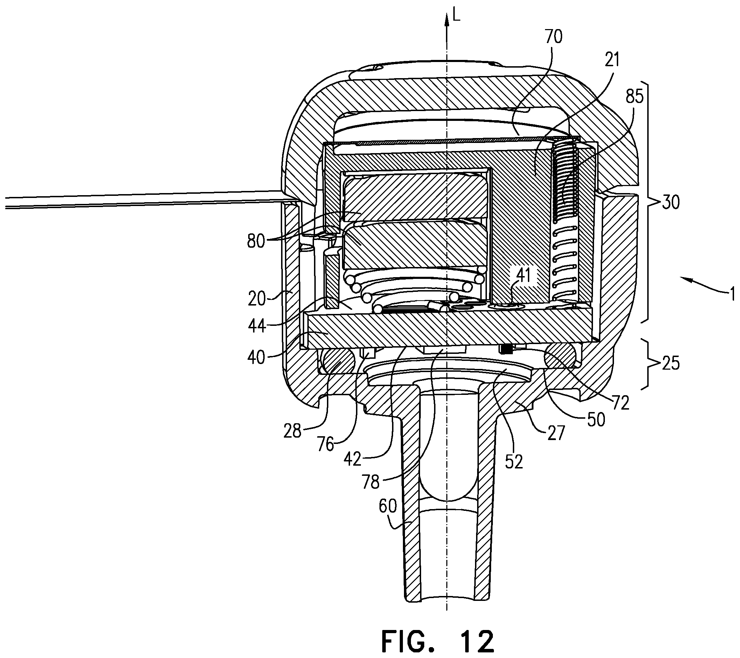

The device **1** further comprises a pressure sensor **72** disposed at the first face **42** of the PCB **40** and being in fluid communication with the fluid-tight volume **52** of the fluid chamber **50** for measuring fluid pressure at the fluid chamber **50** being correlative with fluid pressure at the distal end **5'** of the needle **5.** The pressure sensor **72** is configured to be affected by the pressure of the fluid within the fluid-tight volume **52.** The pressure sensor **72** is a gauge pressure sensor, which utilizes the ambient atmospheric pressure as reference. According to the present example, the second face **44** of the PCB **40,** is facing an ambient atmosphere outside the fluid-tight volume **52,** and the gauge pressure sensor is vented by an opening **41** extending through the PCB 40 (shown in **Fig. 12**)**.** The opening **41** provides fluid connection between the gauge pressure sensor and the ambient air, while the pressure sensor **72** prevents fluid connection between the fluid-tight volume **52** and the ambient air.

The device **1** further has a light emitter in the form of an LED **76** disposed at the first face **42** of the PCB **40** and configured to emit light towards and through the light-permeable external wall **27.** The device **1** further has a control unit **78** disposed at the first face **42** of the PCB **40** and configured to: receive pressure measurements from the pressure sensor **72;** analyze the pressure measurements; and according to the analysis of the pressure measurements, activate the LED **76** to emit light through the light-permeable external wall **27.** The control unit **78** may comprise a digital processor executing a suitable program, digital circuitry, analog circuitry, mixed signal circuitry, and/or any suitable combination thereof.

The housing opening **60** has a tubular shape extending at a lowermost end of the housing distal portion and constitutes part of the light-permeable external wall **27,** allowing them to guide the light emitted by the LED **76** to the skin surrounding the blood vessel. This can enable the practitioner of the device not only receive information regarding the state of the distal end of the probe (by the predetermined pattern of illumination), but also provide him lightening to better see the surrounding of the blood vessel during the procedure. It is appreciated that according to the present example, the proximal portion of the probe is made of a light-permeable material as well, allowing it to receive light from the housing opening **60** and illuminate the surrounding of the probe and the patient's skin at the area of the blood vessel.

The device 1 is operative so that upon introduction of the distal end **5'** of the probe into the skin 2 of the patient, the air that resides between the distal end **5'** of the probe and the fluid chamber **50** is being substantially confined therein. Upon further introduction the probe **4** into the blood vessel **2'** and its perforation, blood starts filling the volume between the distal end **5'** of the probe and the fluid chamber **50,** causing the pressure sensor **72** of the device to sense pressure increase of the confined air. This increase is detected by the control unit **78,** which in turn, will cause the LED **76** to emit light at a predetermined pattern that will be understood by the practitioner as an in-vessel state. In case the practitioner will mistakenly further introduce the probe into the vessel, and will perforate another side of the vessel's wall, the control unit will be able to detect this event, and accordingly active the LED **76** to emit light according to another predetermined pattern, associated with the out-of-vessel.

The light signal generated by the LED **76** may comprise light pulses, and the duration or the repetition rate of the light pulses may indicate the pressure values (e.g. higher repletion rate may indicate an in-vessel state). In some cases, for example, the light signal may comprise various colors, and the color may indicate the needle location state. However, these examples are non-limiting, and any other method of indicating the needle location state by varying the light signal may be utilized. It is noted that the fluid pressure at the distal end **5'** of the needle **5** depends on the location of the distal end relative to blood vessel, and therefore the pressure values, which are indicative of the fluid pressure at the distal end may be indicative of the location thereof. In some cases, for example, the blood pressure within the vessel **2** is expected to be higher than the interstitial fluid outside the vessel **2.** Therefore, a light signal indicating lower (higher) pressure may be interpreted by the practitioner as indicating that the distal end is likely to reside outside (inside) the lumen of the vessel, respectively. However, this example is non-limiting, and the practitioner may utilize, additionally or alternatively, other methods for deducing the location of the distal end based the light signal. practitioner

The device **1** is configured to signal to the practitioner regarding the position of the distal end **5'** of the probe **4** while being introduced into the skin of the patient, in order to allow the practitioner to properly position the distal end **5'** within the blood vessel (a vein or an artery) of the patient, and to know whether the distal end **5'** is positioned within the blood vessel or outside the blood vessel. This signaling is performed by the LED **76,** emitting light **76'** (shown in **Fig. 11**) though the light-permeable external wall **27** towards the area between the device 1 and the skin of the patient. This manner of signaling allows providing to the practitioner's eyes **77** visual convenient indication without the need for accurately observing the device or some parts thereof (e.g., a screen on the device), and enabling him to focus his eyes **77** on the introduction site at skin of the patient. The device **1** is thus configured to be used to avoid "double-puncture". Therefore, obtaining indication regarding the position of the probe relative to the blood vessel **2'** may assist the practitioner in accurately performing the vascular access procedure.

The control unit **78** is configured to determine, based on the analysis of the pressure measurements, a state indicative of the of the position of the distal end **5'** of the probe relative to the blood vessel **2'** of the patient, and to activate the LED **76** to emit light at a predetermined pattern associated with the state. The state can be one of: an in-vessel state indicating that the distal end **5'** is likely to reside within the blood vessel **2',** and an out-of-vessel state indicating that the distal end **5'** is likely to reside out of the blood vessel **2'.** The predetermined pattern of each one of the states can be determined by the number or frequency of light blinks generated by the LED **76.**

In some examples, the state may be indicated by a presence or an absence of emitted light. For example, the "in-vessel" state may be indicated by a continuous emission of light, and the "out-of-vessel" state may be indicated by an absence of emitted light. In some embodiments, the state may be indicated by a modulation of the emitted light. For example, the "in-vessel" state may be indicated by a continuous emission of light, and the "out-of-vessel" state may be indicated by switching the emitted light on and off. It should be appreciated, though, that the above cases are brought here as non-limiting examples, and other methods for indicating the state by controlling the emitted light, for example by controlling the color of the emitted light, or any other method suitable for the case, may be utilized.

The control unit **78** may determine the state by comparing the pressure values with one or more thresholds and setting the state accordingly. In some cases, for example, the state processor may set the state to the in-vessel state when the pressure-values are above a first threshold, and/or set the state to the out-of-vessel state when the pressure-values are below a second threshold. In case where the blood vessel is a vein, the first and second thresholds may be, for example, 15 and 5 millibar, respectively. It is noted, however, that those examples are non-limiting, and the state processor may determine the state by utilizing different thresholds, and/or by utilizing other methods, for example methods based on detecting fluctuations of the blood pressure, and/or any other method suitable for the case.

According to other examples, the LED **76** may be configured to produce light at various colors, associated with the different predetermined patterns of illuminations related to different states of the distal end **5'.** In some cases, for example, the LED **76** may comprise plurality of light sources, wherein different sources are configured to produce light at different colors. In some cases, for example, the LED 76 may vary the color of the emitted light by varying the relative intensity of the light sources.

Perceiving the visual position indication produced by the device **1** may assist the practitioner in performing the vascular access procedure. In some cases, the visual position indication can be perceived by observing the device **1,** and the practitioner may perceive the visual position indication by observing the device while performing the procedure. In some cases, for example, the light-permeable external wall **27** may be transparent, thereby enabling the light emitted by the emitter to pass therethrough and to reach the eyes of the practitioner directly, without being scattered. As shown in **Fig. 11****,** the probe has a scattering member **9,** allowing the light emitted by the LED to scattered and reach the eyes of the practitioner.

Is should be appreciated, though, that in some cases the practitioner may prefer to perform the procedure while holding the probe in a posture that causes the device **1** to be hidden from his sight. For example, the practitioner may grasp the device **1** and/or the proximal end of the probe while covering the device **1** with his fingers and/or with its palm. It should be further appreciated that while performing the procedure, the practitioner may tend to focus his visual attention on the probe and on the patient, and especially on the location where the distal end of the probe penetrates the skin of the patient. Therefore, even in cases where the device is not hidden from his eyes, the practitioner may shift his visual attention away from the device.

The device **1** can further comprises an acoustic emitter, in the form of a buzzer **70** located at an upper end of the housing proximal portion **30** and configured to produce acoustic signals indicative of the location of the distal end **5'** of the needle **5.** According to this structure of the device, the LED **76** and the buzzer **70** are positioned at opposite sides of the device 1, while the location of each one of them is optimally positioned with respect to its functionally. In other words, the LED **76** is located at the housing distal portion **25** in order to properly illuminate the area of the probe **4** without any disturbance (e.g., by the hand of the practitioner holding the device) to the line of sight of other parts of the device **1,** and the buzzer is located at the housing proximal portion **30** so be as close as possible and in direct vector to the ears of the practitioner. The buzzer **70** comprises a piezoelectric transducer configured to convert oscillating electric signal into audio signal. However, this example is non-limiting, and other acoustic emitters, such as a buzzer comprising mechanical and/or an electromechanical transducer, and/or any other audio signaling device suitable for the case, may be utilized.

The control unit **78** is configured, based on the analysis of the pressure measurements, to activate the buzzer **70** to produce the acoustic signals towards the practitioner. The control unit **78** is configured, based on the analysis of the pressure measurements, to determine a state indicative of the position of the distal end **5'** of the needle **5** relative to the blood vessel **2'** of the patient, and to activate the buzzer **70** to produce the acoustic signals at a predetermined pattern associated with the state.

The control unit **78** is configured to activate the LED **76** simultaneously to the activation of the buzzer **70.** The activation of the LED **76** and the buzzer **70** at the same time allows these two emitters to compensate the operation of each other. For example, when the surrounding of the procedure is noisy, the LED **76** compensates the operation of the buzzer **70.** According to another example, when there are some difficulties for observing the surrounding the blood vessel **2** and the light emitted by the LED 76, the buzzer **70** can assist the operation to properly detect the state of the distal end **5'** of the needle **5.** The simultaneous activation of the buzzer **70** and the LED **76** can be performed according to the same predetermined pattern.

In some cases, for example, the frequency of the acoustic signal of the buzzer **70** may be indicative of the pressure (e.g., higher frequency indicating higher pressure). However, this example is non-limiting, and the pressure may be indicated by the acoustic signal in other methods, for example by acoustic pulses whose repetition rate indicates the pressure, and/or by any other method suitable for the case.

The device **1** further comprise a power supply component in the form of batteries 80 positioned between the second face **44** of the PCB **40** and the buzzer **70.**

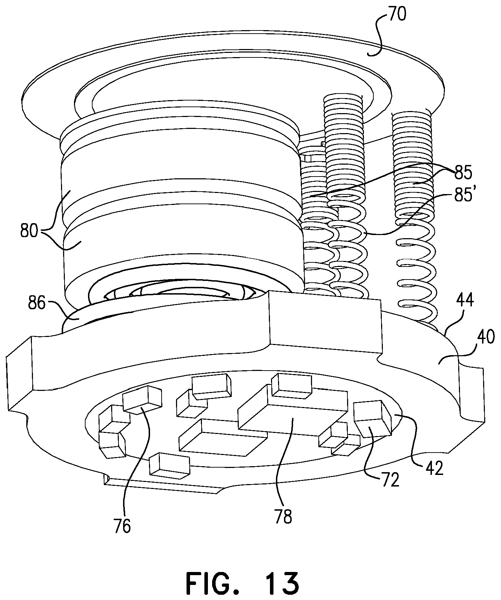

As best seen in **Figs. 12** and **13****,** the buzzer **70** has a flattened shape extending over a majority of a cross section of the housing proximal portion **30** taken perpendicularly to the longitudinal axis L of the housing. This structure of the buzzer **70** allows it on one hand to emit strong enough signals, and one the other hand to be compactly accommodated within the housing **20.**

The device **1** further comprises two electrically conductive metal helical springs **85** providing galvanic connection between the second face **44** of the PCB **40** and the buzzer **70,** thereby enabling delivery of electric power and/or control from the PCB **40** to the buzzer **70.** The spring **85** are galvanically connected to contacts points located on the PCB **40** and contact points of the buzzer **70.** The springs **85** engage the contact points, and the contact required to ensure galvanic connectivity is obtained due to the pressure applied by the springs **85.** It is appreciated this implementation of electric connectivity facilitates easier assembly of the device 1 and reduction of the size of the device 1, and that the springs **85** are not attached to the contact points (i.e., not wired, not soldered).

The batteries **80** are also in galvanic connection with the PCB **40.** As illustrated in **Fig. 13****,** a negative terminal of a lower one of the batteries **80** is galvanically connected to a contact point on the PCB **40** through an elastic conductive member **86,** such as a metal. However, the opposite positive terminal of one upper one of the batteries 80 is spaced from the PCB **40,** and its connection to the PCB **40** is provided by an additional electrically conductive spring **85',** which provides galvanic connection between the PCB 40 and the upper battery. As shown in **Fig. 12****,** the batteries **80** and the springs **85** and **85'** are held by a sub-housing **21** positioned in the housing proximal portion **30.** The sub-housing **21** comprises a cavity for accommodating the batteries **80** bores for holding the springs **85** and **85'.**

In this description, references to "one embodiment" or "an embodiment" mean that the feature being referred to may be included in at least one embodiment of the invention. Moreover, separate references to "one embodiment" or "an embodiment" or "some embodiments" in this description do not necessarily refer to the same embodiment. Additionally, references to "one embodiment", "an embodiment", and "another embodiment" may not necessarily refer to different embodiments, but may be terms used, at times, to illustrate different aspects of an embodiment.

The embodiments of the invention may include any variety of combinations and/or integrations of the features of the embodiments described herein. Although some embodiments may depict serial operations, the embodiments may perform certain operations in parallel and/or in a different order from those depicted. Moreover, the use of repeated reference numerals and/or letters in the text and/or drawings is for the purpose of simplicity and clarity and does not dictate a relationship between the various embodiments and/or configurations discussed. The embodiments are not limited in their applications to the details of the order or sequence of steps of operation of methods, or to details of implementation of devices, set in the description, drawings, or examples. Moreover, individual blocks illustrated in the figures may be functional in nature and therefore may not necessarily correspond to discrete hardware elements.

While the methods disclosed herein have been described and shown with reference to particular steps performed in a particular order, it is understood that these steps may be combined, sub-divided, and/or reordered to form an equivalent method without departing from the teachings of the embodiments. Accordingly, unless specifically indicated herein, the order and grouping of the steps is not a limitation of the embodiments. Furthermore, methods and mechanisms of the embodiments will sometimes be described in singular form for clarity. However, some embodiments may include multiple iterations of a method or multiple instantiations of a mechanism, unless noted otherwise. For example, when a processor is disclosed in an embodiment, the scope of the embodiment is intended also to cover the use of multiple processors. Certain features of the embodiments, which may have been, for clarity, described in the context of separate embodiments, may also be provided in various combinations in a single embodiment. Conversely, various features of the embodiments, which may have been, for brevity, described in the context of a single embodiment, may also be provided separately, or in any suitable sub-combination. Embodiments described in conjunction with specific examples are presented by way of example, and not limitation.

## Claims

1. A device to be used with a probe having a distal end and defining a lumen, the device comprising a control unit being configured and operable to receive a first pressure pattern comprising a plurality of pressure values; the first pressure pattern being indicative of a pressure of a fluid when the distal end of the probe is located inside a blood vessel; processing the first pressure pattern and determining at least one adaptive threshold and/or a tendency indication; wherein the adaptive threshold is indicative of a transition from a first position state to a second position state or *vice versa* and the tendency indication is indicative of a certain trend of the pressure pattern, wherein the first position state defines an in-blood vessel condition in which the distal end of the probe is located inside the blood vessel and the second position state defines at least one exit condition in which the distal end of the probe is located at least partially outside the blood vessel; **characterized in that** said control unit is configured and operable for updating said adaptive threshold based on new pressure values to define a monotonically increasing threshold function over time.

2. The device of claim 1, wherein said control unit is configured and operable to obtain at least one pressure-value being indicative of a certain position state, comparing the at least one pressure value of the first pressure pattern to the adaptive threshold; determining whether the at least one pressure-value is below/above the adaptive threshold; and indicating a position state of a distal end of a probe relative to a blood vessel, wherein, if the distal end of the probe is in the first position state, and the pressure value is above the adaptive threshold, the position state corresponds to the first position state, and if the pressure value is below the adaptive threshold, the position state corresponds to the second position state, and wherein, if the distal end of the probe is in the second position state, and the pressure value is above the adaptive threshold, the position state corresponds to the first position state, and if the pressure value is below the adaptive threshold, the position state corresponds to the second position state.

3. The device of claim 1 or 2, wherein said control unit is configured and operable to determine at least one adaptive threshold by identifying, in the first pressure pattern, at least one special pressure value being indicative of a certain parameter, and defining the at least one adaptive threshold to correspond to the at least one special pressure value or to a function of a plurality of special pressure values.

4. The device of claim 3, wherein the at least one special pressure value comprises the largest pressure value or the lowest pressure value.

5. The device of any one of claims 1 to 4, wherein, after having determined the at least one adaptive threshold, said control unit is configured and operable to receive a subsequent pressure pattern being indicative of a pressure of a fluid when the distal end of the probe is located inside the blood vessel and updating the value of the at least one adaptive threshold accordingly.

6. The device of any one of claims 1 to 5, wherein the second position state comprises one exit state being indicative of a position in which the distal end of the probe is located outside the blood vessel and first and second intermediate states being indicative of a fast change in pressure as compared to other pressure changes in the pressure pattern, the first intermediate state being indicative of a potential entry into the blood vessel and the second intermediate state being indicative of a potential exit out of the blood vessel.

7. The device of claim 1, wherein said control unit is configured and operable to obtain a tendency condition being indicative of a certain position state; compare the tendency condition to the tendency indication; determine whether the tendency indication meets the tendency condition; and indicate a position state of a distal end of a probe relative to a blood vessel.

8. The device of claim 7, wherein said control unit is configured and operable to receive a second pressure pattern comprising a plurality of pressure values being indicative of a pressure of a fluid when the distal end of the probe is located outside the blood vessel.

9. The device of any one of claims 1 to 8, wherein, when the probe is in the first position state, the tendency indication is indicative of a tendency decreasing over time.

10. The device of any one of claims 1 to 8, wherein, when the probe is in the second position state, the tendency indication is indicative of a tendency increasing over time.

11. The device of any one of claims 1 to 10, further comprising a sensing module being configured and operable to measure pressure values being indicative of a pressure of a fluid at the distal end of the probe.

12. The device of any one of claims 1 to 11, wherein when the probe comprises a needle and a cannula mounted over the needle; said control unit is configured and operable to indicate a position state of a distal end of a probe relative to a blood vessel, wherein the position states include a third position state in which the needle has been withdrawn relative to the cannula, leaving the cannula within the blood vessel, and a fourth position state in which the distal end of the needle has exited the blood vessel.

13. The device of any one of claims 1 to 12, further comprising an accelerometer being configured and operable to measure a direction of a movement from a first position state to a second position state or *vice versa* and a transition force from a first position state to a second position state or *vice versa* and to generate a direction signal being indicative of the transition direction and force.

## Patentansprüche

1. Vorrichtung, die mit einer Sonde verwendet werden soll, die ein distales Ende aufweist und ein Lumen definiert, wobei die Vorrichtung eine Steuereinheit aufweist, die konfiguriert und betriebsfähig ist, ein erstes Druckmuster zu empfangen, das mehrere Druckwerte aufweist; wobei das erste Druckmuster einen Druck eines Fluids anzeigt, wenn sich das distale Ende der Sonde innerhalb eines Blutgefäßes befindet; das erste Druckmuster zu verarbeiten und mindestens einen adaptiven Schwellenwert und/oder eine Tendenzanzeige zu bestimmen; wobei der adaptive Schwellenwert einen Übergang von einem ersten Positionszustand zu einem zweiten Positionszustand oder umgekehrt anzeigt und die Tendenzanzeige einen bestimmten Trend des Druckmusters anzeigt, wobei der erste Positionszustand einen Zustand innerhalb des Blutgefäßes definiert, in dem sich das distale Ende der Sonde innerhalb des Blutgefäßes befindet, und der zweite Positionszustand mindestens einen Austrittszustand definiert, in dem sich das distale Ende der Sonde zumindest teilweise außerhalb des Blutgefäßes befindet;
**dadurch gekennzeichnet, dass**
die Steuereinheit konfiguriert und betriebsfähig ist, um den adaptiven Schwellenwert basierend auf neuen Druckwerten zu aktualisieren, um eine monoton zunehmende Schwellenwertfunktion über die Zeit zu definieren.

2. Vorrichtung nach Anspruch 1, wobei die Steuereinheit konfiguriert und betriebsfähig ist, um mindestens einen Druckwert zu erhalten, der einen bestimmten Positionszustand anzeigt, den mindestens einen Druckwert des ersten Druckmusters mit dem adaptiven Schwellenwert zu vergleichen; zu bestimmen, ob der mindestens eine Druckwert unter/über dem adaptiven Schwellenwert liegt; und einen Positionszustand eines distalen Endes einer Sonde relativ zu einem Blutgefäß anzuzeigen, wobei, wenn sich das distale Ende der Sonde in dem ersten Positionszustand befindet und der Druckwert über dem adaptiven Schwellenwert liegt, der Positionszustand dem ersten Positionszustand entspricht, und wenn der Druckwert unter dem adaptiven Schwellenwert liegt, der Positionszustand dem zweiten Positionszustand entspricht, und wobei, wenn sich das distale Ende der Sonde im zweiten Positionszustand befindet und der Druckwert über dem adaptiven Schwellenwert liegt, der Positionszustand dem ersten Positionszustand entspricht, und wenn der Druckwert unter dem adaptiven Schwellenwert liegt, der Positionszustand dem zweiten Positionszustand entspricht.

3. Vorrichtung nach Anspruch 1 oder 2, wobei die Steuereinheit konfiguriert und betriebsfähig ist, um mindestens einen adaptiven Schwellenwert zu bestimmen, indem sie in dem ersten Druckmuster mindestens einen speziellen Druckwert identifiziert, der einen bestimmten Parameter anzeigt, und den mindestens einen adaptiven Schwellenwert so definiert, dass er dem mindestens einen speziellen Druckwert oder einer Funktion von mehreren speziellen Druckwerten entspricht.

4. Vorrichtung nach Anspruch 3, wobei der mindestens eine spezielle Druckwert den größten Druckwert oder den niedrigsten Druckwert aufweist.

5. Vorrichtung nach einem der Ansprüche 1 bis 4, wobei die Steuereinheit, nachdem sie den mindestens einen adaptiven Schwellenwert bestimmt hat, konfiguriert und betriebsfähig ist, ein nachfolgendes Druckmuster zu empfangen, das einen Druck eines Fluids anzeigt, wenn sich das distale Ende der Sonde innerhalb des Blutgefäßes befindet, und den Wert des mindestens einen adaptiven Schwellenwertes entsprechend zu aktualisieren.

6. Vorrichtung nach einem der Ansprüche 1 bis 5, wobei der zweite Positionszustand einen Austrittszustand, der eine Position anzeigt, in der sich das distale Ende der Sonde außerhalb des Blutgefäßes befindet, und einen ersten und einen zweiten Zwischenzustand aufweist, die eine schnelle Druckänderung im Vergleich zu anderen Druckänderungen in dem Druckmuster anzeigen, wobei der erste Zwischenzustand einen möglichen Eintritt in das Blutgefäß anzeigt und der zweite Zwischenzustand einen möglichen Austritt aus dem Blutgefäß anzeigt.

7. Vorrichtung nach Anspruch 1, wobei die Steuereinheit konfiguriert und betreibbar ist, um eine Tendenzbedingung zu erhalten, die einen bestimmten Positionszustand anzeigt; die Tendenzbedingung mit der Tendenzanzeige zu vergleichen; zu bestimmen, ob die Tendenzanzeige die Tendenzbedingung erfüllt; und einen Positionszustand eines distalen Endes einer Sonde relativ zu einem Blutgefäß anzuzeigen.

8. Vorrichtung nach Anspruch 7, wobei die Steuereinheit konfiguriert und betriebsfähig ist, um ein zweites Druckmuster zu empfangen, das mehrere Druckwerte aufweist, die einen Druck eines Fluids anzeigen, wenn sich das distale Ende der Sonde außerhalb des Blutgefäßes befindet.

9. Vorrichtung nach einem der Ansprüche 1 bis 8, wobei, wenn sich die Sonde im ersten Positionszustand befindet, die Tendenzanzeige eine mit der Zeit abnehmende Tendenz anzeigt.

10. Vorrichtung nach einem der Ansprüche 1 bis 8, wobei, wenn sich die Sonde im zweiten Positionszustand befindet, die Tendenzanzeige eine mit der Zeit zunehmende Tendenz anzeigt.

11. Vorrichtung nach einem der Ansprüche 1 bis 10, die ferner ein Sensormodul aufweist, das konfiguriert und betriebsfähig ist, um Druckwerte zu messen, die einen Druck eines Fluids am distalen Ende der Sonde anzeigen.

12. Vorrichtung nach einem der Ansprüche 1 bis 11, wobei, wenn die Sonde eine Nadel und eine über der Nadel angebrachte Kanüle aufweist, die Steuereinheit konfiguriert und betriebsfähig ist, um einen Positionszustand eines distalen Endes einer Sonde relativ zu einem Blutgefäß anzuzeigen, wobei die Positionszustände einen dritten Positionszustand, in dem die Nadel relativ zur Kanüle zurückgezogen wurde, wobei die Kanüle im Blutgefäß belassen wurde, und einen vierten Positionszustand umfassen, in dem das distale Ende der Nadel das Blutgefäß verlassen hat.

13. Vorrichtung nach einem der Ansprüche 1 bis 12, die ferner einen Beschleunigungsmesser aufweist, der konfiguriert und betriebsfähig ist, um eine Bewegungsrichtung von einem ersten Positionszustand zu einem zweiten Positionszustand oder umgekehrt und eine Übergangskraft von einem ersten Positionszustand zu einem zweiten Positionszustand oder umgekehrt zu messen und ein Richtungssignal zu erzeugen, das die Übergangsrichtung und -kraft anzeigt.

## Revendications

1. Dispositif destiné à être utilisé avec une sonde ayant une extrémité distale et définissant une lumière, ledit dispositif comprenant une unité de commande configurée, et utilisable pour recevoir un premier motif de pression comprenant une pluralité de valeurs de pression ; ledit premier motif de pression étant indicatif de la pression d'un fluide lorsque l'extrémité distale de la sonde est présentée à l'intérieur d'un vaisseau sanguin ; pour traiter le premier motif de pression et déterminer au moins un seuil adaptatif et/ou une indication de tendance ; où le seuil adaptatif est indicatif d'une transition d'un premier état de position à un deuxième état de position ou inversement, et l'indication de tendance est indicative d'une certaine tendance du motif de pression, où le premier état de position définit une condition dans le vaisseau sanguin où l'extrémité distale de la sonde est présentée à l'intérieur du vaisseau sanguin, et le deuxième état de position définit au moins une condition de sortie où l'extrémité distale de la sonde est présentée au moins partiellement en dehors du vaisseau sanguin ;
**caractérisé en ce que**
l'unité de commande est configurée et utilisable pour mettre à jour le seuil adaptatif sur la base de nouvelles valeurs de pression afin de définir une fonction de seuil à croissance monotone dans le temps.

2. Dispositif selon la revendication 1, où l'unité de commande est configurée et utilisable pour obtenir au moins une valeur de pression indicative d'un certain état de position, pour comparer ladite au moins une valeur de pression du premier motif de pression au seuil adaptatif ; pour déterminer si ladite au moins une valeur de pression est inférieure/supérieure au seuil adaptatif ; et pour indiquer un état de position d'une extrémité distale d'une sonde par rapport à un vaisseau sanguin, où, si l'extrémité distale de la sonde se trouve dans le premier état de position, et que la valeur de pression est supérieure au seuil adaptatif, l'état de position correspond au premier état de position, et, si la valeur de pression est inférieure au seuil adaptatif, l'état de position correspond au deuxième état de position, et où, si l'extrémité distale de la sonde se trouve dans le deuxième état de position, et que la valeur de pression est supérieure au seuil adaptatif, l'état de position correspond au premier état de position, et si la valeur de pression est inférieure au seuil adaptatif, l'état de position correspond au deuxième état de position.

3. Dispositif selon la revendication 1 ou 2, où l'unité de commande est configurée et est utilisable pour déterminer au moins un seuil adaptatif en identifiant, dans le premier motif de pression, au moins une valeur de pression spécifique indicative d'un certain paramètre, et définit ledit au moins un seuil adaptatif de manière à ce qu'il corresponde à ladite au moins une valeur de pression spécifique ou à une fonction d'une pluralité de valeurs de pression spécifiques.

4. Dispositif selon la revendication 3, où ladite au moins une valeur de pression spécifique comprend la valeur de pression maximale ou la valeur de pression minimale.

5. Dispositif selon l'une des revendications 1 à 4, où, après détermination dudit au moins un seuil adaptatif, l'unité de commande est configurée et utilisable pour recevoir un motif de pression ultérieur indiquant la pression d'un fluide lorsque l'extrémité distale de la sonde est présentée à l'intérieur du vaisseau sanguin et pour mettre à jour la valeur dudit au moins un seuil adaptatif en conséquence.

6. Dispositif selon l'une des revendications 1 à 5, où le deuxième état de position comprend un état de sortie indiquant une position où l'extrémité distale de la sonde est présentée en dehors du vaisseau sanguin, et un premier et un deuxième états intermédiaires indiquant une variation subite de pression par rapport à d'autres variations de pression dans le motif de pression, le premier état intermédiaire indiquant une entrée potentielle dans le vaisseau sanguin et le deuxième état intermédiaire indiquant une sortie potentielle hors du vaisseau sanguin.

7. Dispositif selon la revendication 1, où l'unité de commande est configurée et utilisable pour obtenir une condition de tendance indiquant un certain état de position ; pour comparer la condition de tendance à l'indication de tendance ; pour déterminer si l'indication de tendance satisfait à la condition de tendance ; et pour indiquer un état de position d'une extrémité distale d'une sonde par rapport à un vaisseau sanguin.

8. Dispositif selon la revendication 7, où l'unité de commande est configurée et utilisable pour recevoir un deuxième motif de pression comprenant une pluralité de valeurs de pression indiquant la pression d'un fluide lorsque l'extrémité distale de la sonde est présentée en dehors du vaisseau sanguin.

9. Dispositif selon l'une des revendications 1 à 8, où, lorsque la sonde se trouve dans le premier état de position, l'indication de tendance indique une tendance à décroître dans le temps.

10. Dispositif selon l'une des revendications 1 à 8, où, lorsque la sonde se trouve dans l'état de deuxième position, l'indication de tendance indique une tendance à croître dans le temps.

11. Dispositif selon l'une des revendications 1 à 10, comprenant en outre un module de détection configuré et utilisable pour mesurer des valeurs de pression indiquant la pression d'un fluide à l'extrémité distale de la sonde.

12. Dispositif selon l'une des revendications 1 à 11, où, lorsque la sonde comprend une aiguille et une canule montée sur l'aiguille, l'unité de commande est configurée et utilisable pour indiquer un état de position d'une extrémité distale d'une sonde par rapport à un vaisseau sanguin, où les états de position comprennent un troisième état de position où l'aiguille a été retirée par rapport à la canule, laissant la canule à l'intérieur du vaisseau sanguin, et un quatrième état de position où l'extrémité distale de l'aiguille est sortie du vaisseau sanguin.

13. Dispositif selon l'une des revendications 1 à 12, comprenant en outre un accéléromètre configuré et utilisable pour mesurer une direction de déplacement d'un premier état de position à un deuxième état de position ou inversement, ainsi qu'une force de transition d'un premier état de position à un deuxième état de position ou inversement, et pour générer un signal de direction indiquant la direction et la force de transition.
